# EUROPEAN PATENT APPLICATION

(11) **EP 4 056 592 A1**
(43) Date of publication of application: **14.09.2022**
(21) Application number: 20884635.2
(22) Date of filing: 05.11.2020
(51) Int. Cl.: C07K 16/28, C12N 15/13, C12P 21/00

(54) **ANTI-HUMAN PROGRAMMED CELL DEATH LIGAND-1 (PD-L1) ANTIBODY AND USE THEREOF**

(30) Priority: 08.11.2019 CN 201911088643
(71) Applicant: Simcere (Shanghai) Pharmaceutical Co., Ltd., Shanghai 201321 (CN); Jiangsu Simcere Pharmaceutical Co., Ltd., Nanjing, Jiangsu 210042 (CN)
(72) Inventor: ZHAO, Xinyan, Nanjing, Jiangsu 210042 (CN); DENG, Jing, Nanjing, Jiangsu 210042 (CN); LU, Shiqiang, Nanjing, Jiangsu 210042 (CN); LI, Xinxin, Nanjing, Jiangsu 210042 (CN); REN, Jinsheng, Nanjing, Jiangsu 210042 (CN)
(74) Representative: Lavoix
(86) International application number: PCT/CN2020/126656
(87) International publication number: WO 2021/088904

(57) **Abstract**

The present invention relates to an antibody or antigen-binding fragment specifically binding to human programmed cell death ligand-1 (PD-L1), the antibody or antigen-binding fragment being able to enhance the function of T cells and upregulate a T cell-mediated immune response; the invention also relates to use of the antibody or the antigen-binding fragment for the treatment of diseases, e.g. tumor, associated with aberrant expression of PD-L1 and/or dysfunction of T cells.

SEQUENCE LISTING

<110>  SIMCERE BIO-PHARMACEUTICAL TECHNOLOGY., LTD.

<120>  ANTI-HUMAN PROGRAMMED CELL DEATH LIGAND-1 (PD-L1) ANTIBODY AND
       USE THEREOF

<130>  Unitalen 1639

<150>  CN 201911088643.5
<151>  2019-11-08

<150>  PCT/CN2020/126656
<151>  2020-11-05

<160>  182

<170>  PatentIn version 3.5

<210>  1
<211>  118
<212>  PRT
<213>  Artificial Sequence

<220>
<223>  Heavy chain variable region

<400>  1

Glu Val Gln Leu Gln Glu Ser Gly Gly Asp Leu Val Lys Pro Gly Gly
1               5                   10                  15

Ser Leu Lys Leu Ser Cys Ala Ala Ser Gly Phe Thr Phe Asn Thr Tyr
            20                  25                  30

Gly Met Ser Trp Val Arg Gln Thr Pro Asp Lys Arg Leu Glu Trp Val
        35                  40                  45

Ala Thr Leu Ser Ser Gly Gly Ser Tyr Thr Tyr Tyr Ser Glu Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Ile Tyr Tyr Cys
                85                  90                  95

Ala Arg Pro Thr Ala Asp Trp His Leu Asp Val Trp Gly Thr Gly Thr
            100                 105                 110

Thr Val Thr Val Ser Ser
        115

<210>  2
<211>  107

<212>  PRT
<213>  Artificial Sequence

<220>
<223>  Light chain variable region

<400>  2

Asp Ile Leu Met Thr Gln Ser Pro Ser Ser Leu Ser Ala Ser Leu Gly
1               5                   10                  15

Gly Lys Val Thr Ile Thr Cys Asn Ala Ser Gln Asp Ile Asn Lys Tyr
            20                  25                  30

Ile Ala Trp Tyr Gln His Lys Pro Gly Lys Gly Pro Ser Leu Leu Ile
        35                  40                  45

His Tyr Thr Ser Thr Leu Gln Pro Gly Ile Pro Ser Arg Phe Ser Gly
    50                  55                  60

Ser Gly Ser Gly Arg Asp Tyr Ser Phe Ser Ile Ser Asn Leu Glu Pro
65                  70                  75                  80

Glu Asp Ile Ala Thr Tyr Tyr Cys Leu Gln His Asp Asn Leu Leu Phe
                85                  90                  95

Thr Phe Gly Ser Gly Thr Lys Leu Glu Ile Lys
            100                 105


<210>  3
<211>  118
<212>  PRT
<213>  Artificial Sequence

<220>
<223>  Heavy chain variable region

<400>  3

Glu Val Gln Leu Gln Glu Ser Gly Gly Asp Leu Val Lys Pro Gly Gly
1               5                   10                  15

Ser Leu Lys Leu Ser Cys Ala Ala Ser Gly Phe Thr Phe Asn Thr Tyr
            20                  25                  30

Gly Met Ser Trp Val Arg Gln Thr Pro Asp Lys Arg Leu Glu Trp Val
        35                  40                  45

Ala Thr Leu Ser Ser Gly Gly Ser Tyr Thr Tyr Tyr Ser Glu Ser Val
    50                  55                  60

Lys Gly Arg Phe Thr Ile Ser Arg Asp Asn Ala Lys Asn Thr Leu Tyr

65                  70                  75                  80

Leu Gln Met Thr Ser Leu Lys Ser Glu Asp Thr Ala Ile Tyr Tyr Cys
                85                  90                  95

Ala Arg Pro Thr Ala Asp Trp His Leu Asp Val Trp Gly Thr Gly Thr
            100                 105                 110

Thr Val Thr Val Ser Ser
        115

<210>  4
<211>  107
<212>  PRT
<213>  Artificial sequence

<220>
<223>  Light chain variable region

<400>  4

Asp Ile Gln Met Thr Gln Ser Pro Ser Ser Leu Ser Ala Ser Leu Gly
1               5                   10                  15

Gly Lys Val Thr Ile Thr Cys Lys Ala Ser Gln Asp Ile Asn Lys Tyr
            20                  25                  30

Ile Ala Trp Tyr Gln His Lys Pro Gly Lys Gly Pro Arg Leu Leu Ile
        35                  40                  45

His Tyr Thr Ser Thr Leu Gln Pro Gly Ile Pro Ser Arg Phe Ser Gly
    50                  55                  60

Ser Gly Ser Gly Arg Asp Tyr Ser Phe Ser Ile Ser Asn Leu Glu Pro
65                  70                  75                  80

Glu Asp Ile Ala Thr Tyr Tyr Cys Leu Gln His Asp Asn Leu Leu Phe
                85                  90                  95

Thr Phe Gly Ser Gly Thr Lys Leu Glu Ile Lys
            100                 105

<210>  5
<211>  118
<212>  PRT
<213>  Artificial Sequence

<220>
<223>  Heavy chain variable region

<400>  5

Glu Val Gln Leu Gln Glu Ser Gly Gly Asp Leu Val Lys Pro Gly Gly
1               5                   10                  15

Ser Leu Lys Leu Ser Cys Thr Ala Ser Gly Phe Thr Phe Ser Thr Tyr
            20                  25                  30

Gly Met Ser Trp Val Arg Gln Thr Pro Asp Lys Arg Leu Glu Trp Val
        35                  40                  45

Ala Thr Val Ser Ser Gly Gly Ser Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Ile Tyr Tyr Cys
                85                  90                  95

Ala Arg Pro Thr Ala Asp Trp His Leu Asp Val Trp Gly Thr Gly Thr
            100                 105                 110

Thr Val Thr Val Ser Ser
        115

<210>  6
<211>  107
<212>  PRT
<213>  Artificial sequence

<220>
<223>  Light chain variable region

<400>  6

Asp Ile Gln Met Thr Gln Thr Pro Ser Ser Leu Ser Ala Ser Leu Gly
1               5                   10                  15

Gly Lys Val Thr Ile Thr Cys Lys Ala Ser Gln Asp Ile Asn Arg Tyr
            20                  25                  30

Ile Ala Trp Tyr Gln His Lys Pro Gly Lys Gly Pro Ser Leu Leu Ile
        35                  40                  45

His Tyr Thr Ser Thr Leu Gln Pro Gly Ile Pro Ser Arg Phe Ser Gly
    50                  55                  60

Ser Gly Ser Gly Arg Asp Tyr Ser Phe Ser Ile Ser Asn Leu Glu Pro
65                  70                  75                  80

Glu Asp Ile Ala Thr Tyr Tyr Cys Leu Gln His Asp Asn Leu Leu Phe

85                  90                  95

Thr Phe Gly Ser Gly Thr Lys Leu Glu Ile Lys
            100                 105

<210>  7
<211>  118
<212>  PRT
<213>  Artificial Sequence

<220>
<223>  Heavy chain variable region

<400>  7

Glu Val Gln Leu Gln Glu Ser Gly Pro Gly Leu Ala Lys Pro Ser Gln
1               5                   10                  15

Thr Leu Ser Leu Thr Cys Ser Val Thr Gly Tyr Ser Ile Thr Ser Asp
            20                  25                  30

Tyr Trp Asn Trp Ile Arg Lys Phe Pro Gly Asn Lys Leu Glu Tyr Val
        35                  40                  45

Gly Tyr Ile Ser Tyr Thr Gly Ser Thr Tyr Tyr Asn Pro Ser Leu Arg
    50                  55                  60

Ser Arg Ile Ser Ile Thr Arg Asp Thr Ser Lys Asn Gln Tyr Tyr Leu
65                  70                  75                  80

Gln Leu Asn Ser Val Thr Ala Glu Asp Thr Ala Thr Tyr Tyr Cys Ala
                85                  90                  95

Arg Cys Pro Gly Trp Leu Asn Ala Met Asp Tyr Trp Gly Gln Gly Thr
            100                 105                 110

Thr Val Thr Val Ser Ser
        115

<210>  8
<211>  113
<212>  PRT
<213>  Artificial sequence

<220>
<223>  Light chain variable region

<400>  8

Asp Ile Leu Met Thr Gln Ser Pro Ser Ser Leu Ala Val Ser Val Gly
1               5                   10                  15

Glu Lys Val Thr Leu Ser Cys Lys Ser Ser Gln Ser Leu Leu Tyr Ser
            20                  25                  30

Asn Asn Gln Lys Asn Ser Leu Ala Trp Tyr Gln Gln Lys Pro Gly Gln
        35                  40                  45

Ser Pro Lys Leu Leu Ile Tyr Trp Ala Ser Thr Gly Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Gly Tyr Pro Phe Thr Phe Gly Ala Gly Thr Lys Leu Glu Ile
            100                 105                 110

Lys

<210> 9
<211> 114
<212> PRT
<213> Artificial Sequence

<220>
<223> Heavy chain variable region

<400> 9

Glu Val Gln Leu Gln Glu Ser Gly Pro Glu Leu Val Lys Pro Gly Ala
1               5                   10                  15

Ser Val Lys Ile Ser Cys Lys Ala Ser Gly Tyr Thr Phe Thr Asp Tyr
            20                  25                  30

Tyr Met Asn Trp Val Arg Gln Ser His Gly Lys Ser Leu Glu Trp Ile
        35                  40                  45

Gly Asp Ile Asn Pro Asn Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Asp Leu Arg Ser Leu Thr Ser Glu Asp Ser Ala Val Tyr Tyr Cys
                85                  90                  95

Ala Ser Ser Val Met Asp Tyr Trp Gly Gln Gly Thr Thr Val Thr Val

            100                 105                 110

Ser Ser

<210> 10
<211> 106
<212> PRT
<213> Artificial sequence

<220>
<223> Light chain variable region

<400> 10

Asp Ile Val Leu Thr Gln Ser Pro Ala Leu Met Ser Ala Ser Pro Gly
1               5                   10                  15

Glu Lys Val Thr Met Thr Cys Ser Ala Ser Ser Ser Val Asn Tyr Val
            20                  25                  30

Tyr Trp Tyr Gln Gln Lys Pro Arg Ser Ser Pro Lys Pro Trp Ile Tyr
        35                  40                  45

Leu Thr Phe Asn Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Leu Thr
                85                  90                  95

Phe Gly Ala Gly Thr Lys Leu Glu Ile Lys
            100                 105

<210> 11
<211> 118
<212> PRT
<213> Artificial Sequence

<220>
<223> Heavy chain variable region

<400> 11

Glu Val Gln Leu Gln Glu Ser Gly Pro Gly Leu Ala Lys Pro Ser Gln
1               5                   10                  15

Thr Leu Ser Leu Thr Cys Ser Val Thr Gly Tyr Ser Ile Thr Ser Asp
            20                  25                  30

Tyr Trp Asn Trp Ile Arg Lys Phe Pro Gly Asn Lys Leu Glu Tyr Met
        35                  40                  45

Gly Tyr Ile Ser Tyr Thr Gly Ser Thr Tyr Tyr Asn Pro Ser Leu Lys
    50                  55                  60

Ser Arg Ile Ser Ile Ala Arg Asp Thr Ser Arg Asn Gln Tyr Tyr Leu
65                  70                  75                  80

Gln Leu Asn Ser Val Thr Thr Glu Asp Ser Ala Thr Tyr Tyr Cys Ala
                85                  90                  95

Arg Ala Gly Gly Trp Leu Leu Pro Phe Ala Val Trp Gly Thr Gly Thr
            100                 105                 110

Thr Val Thr Val Ser Ser
        115

<210>  12
<211>  113
<212>  PRT
<213>  Artificial sequence

<220>
<223>  Light chain variable region

<400>  12

Asp Ile Val Ile Thr Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Gln Asn Ser Leu Ala Trp Tyr Gln Gln Lys Pro Gly Gln
        35                  40                  45

Ser Pro Ile Leu Leu Ile Tyr Trp Ala Ser Thr Arg Glu Ser Gly Val
    50                  55                  60

Pro Asp Arg Phe Thr Gly Gly Gly Ser Gly Thr Asp Phe Thr Leu Thr
65                  70                  75                  80

Ile Ser Ser Val Arg Ala Glu Asp Leu Ala Val Tyr Tyr Cys Gln Gln
                85                  90                  95

Tyr Tyr Asn Tyr Pro Trp Thr Phe Gly Gly Gly Thr Lys Leu Glu Ile
            100                 105                 110

Lys

<210>  13
<211>  118
<212>  PRT
<213>  Artificial Sequence

<220>
<223>  Heavy chain variable region

<400>  13

Glu Val Gln Leu Gln Glu Ser Gly Gly Gly Leu Val Lys Pro Gly Gly
1               5                   10                  15

Ser Leu Lys Leu Ser Cys Ala Ala Ser Gly Phe Thr Phe Ser Asp Tyr
            20                  25                  30

Gly Met His Trp Val Arg Gln Ala Pro Glu Lys Gly Leu Glu Trp Val
        35                  40                  45

Ala Tyr Ile Ser Ser Gly Ser Ser Thr Ile Tyr Tyr Ala Asp Thr Val
    50                  55                  60

Lys Gly Arg Phe Thr Ile Ser Arg Asp Asn Ala Lys Asn Thr Leu Phe
65                  70                  75                  80

Leu Gln Met Thr Ser Leu Arg Ser Glu Asp Thr Ala Met Tyr Tyr Cys
                85                  90                  95

Ala Arg Arg Asn Phe Gly Ser Ser Tyr Asp Tyr Trp Gly Gln Gly Thr
            100                 105                 110

Thr Val Thr Val Ser Ser
        115

<210>  14
<211>  107
<212>  PRT
<213>  Artificial sequence

<220>
<223>  Light chain variable region

<400>  14

Glu Ile Val Met Thr Gln Ser His Lys Phe Met Ser Thr Ser Val Gly
1               5                   10                  15

Asp Arg Val Ser Ile Thr Cys Lys Ala Ser Gln Asp Val Asp Thr Pro
            20                  25                  30

Val Ala Trp Tyr Gln Gln Lys Pro Gly Gln Ser Pro Arg Leu Leu Ile
        35                  40                  45

Tyr Trp Ala Ser Ile Arg His Thr Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ser Gly Thr Lys Leu Glu Ile Lys
            100                 105

<210>  15
<211>  118
<212>  PRT
<213>  Artificial Sequence

<220>
<223>  Heavy chain variable region

<400>  15

Glu Val Gln Leu Gln Glu Ser Gly Pro Gly Leu Ala Lys Pro Ser Gln
1               5                   10                  15

Thr Leu Ser Leu Thr Cys Ser Val Thr Gly Tyr Ser Ile Thr Ser Asp
            20                  25                  30

Tyr Trp Asn Trp Ile Arg Lys Phe Pro Gly Asn Lys Leu Glu Tyr Met
        35                  40                  45

Gly Tyr Ile Ser Tyr Thr Gly Ser Thr Tyr Tyr Asn Pro Ser Leu Lys
    50                  55                  60

Ser Arg Ile Ser Ile Ala Arg Asp Thr Ser Arg Asn Gln Tyr Tyr Leu
65                  70                  75                  80

Gln Leu Lys Ser Val Thr Thr Glu Asp Thr Ala Thr Tyr Tyr Cys Ala
                85                  90                  95

Arg Ala Gly Gly Trp Leu Leu Pro Phe Ala Val Trp Gly Thr Gly Thr
            100                 105                 110

Thr Val Thr Val Ser Ser
        115

<210>  16

<211>  113
<212>  PRT
<213>  Artificial sequence

<220>
<223>  Light chain variable region

<400>  16

Asp Ile Val Met Thr Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Gln Asn Ser Leu Ala Trp Tyr Gln Gln Lys Pro Gly Gln
        35                  40                  45

Ser Pro Ile Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Arg Ala Glu Asp Leu Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210>  17
<211>  118
<212>  PRT
<213>  Artificial Sequence

<220>
<223>  Heavy chain variable region

<400>  17

Glu Val Gln Leu Gln Glu Ser Gly Pro Ser Leu Val Lys Pro Ser Gln
1               5                   10                  15

Thr Leu Ser Leu Thr Cys Ser Val Thr Gly Asp Ser Ile Thr Ser Gly
            20                  25                  30

Tyr Trp Asn Trp Ile Arg Lys Phe Pro Gly Asn Lys Leu Glu Tyr Met
        35                  40                  45

Gly Tyr Ile Ser Tyr Ser Gly Ser Thr Tyr Tyr Asn Pro Phe Leu Lys
    50                  55                  60

Ser Arg Ile Ser Ile Thr Arg Asp Thr Ser Lys Asn Gln Tyr Tyr Leu
65                  70                  75                  80

Gln Leu Asn Ser Val Thr Thr Glu Asp Thr Ala Thr Tyr Tyr Cys Ala
                85                  90                  95

Lys Met Gly Asp Trp Leu Ala Trp Phe Ala Tyr Trp Gly Gln Gly Thr
            100                 105                 110

Thr Val Thr Val Ser Ser
        115

<210>  18
<211>  113
<212>  PRT
<213>  Artificial sequence

<220>
<223>  Light chain variable region

<400>  18

Glu Ile Val Met Thr Gln Ser Pro Ser Ser Leu Ala Val Ser Val Gly
1               5                   10                  15

Glu Lys Val Thr Leu Ser Cys Lys Ser Ser Gln Ser Leu Leu Tyr Ser
            20                  25                  30

Ser Asn Gln Lys Asn Ser Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Gly Tyr Pro Tyr Thr Phe Gly Gly Gly Thr Lys Leu Glu Ile
            100                 105                 110

Lys

<210>  19

<211>  5
<212>  PRT
<213>  Artificial sequence

<220>
<223>  CDR1-VH

<400>  19

Thr Tyr Gly Met Ser
1               5


<210>  20
<211>  17
<212>  PRT
<213>  Artificial sequence

<220>
<223>  CDR2-VH

<400>  20

Thr Leu Ser Ser Gly Gly Ser Tyr Thr Tyr Tyr Ser Glu Ser Val Lys
1               5                   10                  15


Gly


<210>  21
<211>  9
<212>  PRT
<213>  Artificial sequence

<220>
<223>  CDR3-VH

<400>  21

Pro Thr Ala Asp Trp His Leu Asp Val
1               5


<210>  22
<211>  11
<212>  PRT
<213>  Artificial sequence

<220>
<223>  CDR1-VL

<400>  22

Asn Ala Ser Gln Asp Ile Asn Lys Tyr Ile Ala
1               5                   10


<210>  23
<211>  7
<212>  PRT
<213>  Artificial sequence

<220>
<223>  CDR2-VL

<400>  23

Tyr Thr Ser Thr Leu Gln Pro
1               5


<210>  24
<211>  9
<212>  PRT
<213>  Artificial sequence

<220>
<223>  CDR3-VL

<400>  24

Leu Gln His Asp Asn Leu Leu Phe Thr
1               5


<210>  25
<211>  5
<212>  PRT
<213>  Artificial sequence

<220>
<223>  CDR1-VH

<400>  25

Thr Tyr Gly Met Ser
1               5


<210>  26
<211>  17
<212>  PRT
<213>  Artificial sequence

<220>
<223>  CDR2-VH

<400>  26

Thr Leu Ser Ser Gly Gly Ser Tyr Thr Tyr Tyr Ser Glu Ser Val Lys
1               5                   10                  15


Gly



<210>  27
<211>  9
<212>  PRT
<213>  Artificial sequence

<220>
<223>  CDR3-VH

<400>  27

Pro Thr Ala Asp Trp His Leu Asp Val
1               5

<210>  28
<211>  11
<212>  PRT
<213>  Artificial sequence

<220>
<223>  CDR1-VL

<400>  28

Lys Ala Ser Gln Asp Ile Asn Lys Tyr Ile Ala
1               5                   10

<210>  29
<211>  7
<212>  PRT
<213>  Artificial sequence

<220>
<223>  CDR2-VL

<400>  29

Tyr Thr Ser Thr Leu Gln Pro
1               5

<210>  30
<211>  9
<212>  PRT
<213>  Artificial sequence

<220>
<223>  CDR3-VL

<400>  30

Leu Gln His Asp Asn Leu Leu Phe Thr
1               5

<210>  31
<211>  5
<212>  PRT
<213>  Artificial sequence

<220>
<223>  CDR1-VH

<400>  31

Thr Tyr Gly Met Ser
1               5

<210>  32
<211>  17
<212>  PRT
<213>  Artificial sequence

<220>
<223>  CDR2-VH

<400>  32

Thr Val Ser Ser Gly Gly Ser Tyr Thr Tyr Tyr Pro Asp Ser Val Lys
1               5                   10                  15

Gly

<210>  33
<211>  9
<212>  PRT
<213>  Artificial sequence

<220>
<223>  CDR3-VH

<400>  33

Pro Thr Ala Asp Trp His Leu Asp Val
1               5

<210>  34
<211>  11
<212>  PRT
<213>  Artificial sequence

<220>
<223>  CDR1-VL

<400>  34

Lys Ala Ser Gln Asp Ile Asn Arg Tyr Ile Ala
1               5                   10

<210>  35
<211>  7
<212>  PRT
<213>  Artificial sequence

<220>
<223>  CDR2-VL

<400>  35

Tyr Thr Ser Thr Leu Gln Pro
1               5

<210>  36
<211>  9
<212>  PRT

<213>  Artificial sequence

<220>
<223>  CDR3-VL

<400>  36

Leu Gln His Asp Asn Leu Leu Phe Thr
1               5


<210>  37
<211>  5
<212>  PRT
<213>  Artificial sequence

<220>
<223>  CDR1-VH

<400>  37

Ser Asp Tyr Trp Asn
1               5


<210>  38
<211>  16
<212>  PRT
<213>  Artificial sequence

<220>
<223>  CDR2-VH

<400>  38

Tyr Ile Ser Tyr Thr Gly Ser Thr Tyr Tyr Asn Pro Ser Leu Arg Ser
1               5                   10                  15


<210>  39
<211>  10
<212>  PRT
<213>  Artificial sequence

<220>
<223>  CDR3-VH

<400>  39

Cys Pro Gly Trp Leu Asn Ala Met Asp Tyr
1               5                   10


<210>  40
<211>  17
<212>  PRT
<213>  Artificial sequence

<220>
<223>  CDR1-VL

<400>  40

Lys Ser Ser Gln Ser Leu Leu Tyr Ser Asn Asn Gln Lys Asn Ser Leu
1               5                   10                  15

Ala

<210>  41
<211>  7
<212>  PRT
<213>  Artificial sequence

<220>
<223>  CDR2-VL

<400>  41

Trp Ala Ser Thr Gly Glu Ser
1               5

<210>  42
<211>  9
<212>  PRT
<213>  Artificial sequence

<220>
<223>  CDR3-VL

<400>  42

Gln Gln Tyr Tyr Gly Tyr Pro Phe Thr
1               5

<210>  43
<211>  5
<212>  PRT
<213>  Artificial sequence

<220>
<223>  CDR1-VH

<400>  43

Asp Tyr Tyr Met Asn
1               5

<210>  44
<211>  17
<212>  PRT
<213>  Artificial sequence

<220>
<223>  CDR2-VH

<400>  44

Asp Ile Asn Pro Asn Asn Gly Asp Thr Ser Tyr Asn Gln Lys Phe Lys
1               5                   10                  15

Gly

<210> 45
<211> 6
<212> PRT
<213> Artificial sequence

<220>
<223> CDR3-VH

<400> 45

Ser Ser Val Met Asp Tyr
1               5

<210> 46
<211> 10
<212> PRT
<213> Artificial sequence

<220>
<223> CDR1-VL

<400> 46

Ser Ala Ser Ser Ser Val Asn Tyr Val Tyr
1               5                   10

<210> 47
<211> 7
<212> PRT
<213> Artificial sequence

<220>
<223> CDR2-VL

<400> 47

Leu Thr Phe Asn Leu Ala Ser
1               5

<210> 48
<211> 9
<212> PRT
<213> Artificial sequence

<220>
<223> CDR3-VL

<400> 48

Gln Gln Trp Ser Ser Asn Pro Leu Thr
1               5

<210> 49
<211> 5

<212>  PRT
<213>  Artificial sequence

<220>
<223>  CDR1-VH

<400>  49

Ser Asp Tyr Trp Asn
1               5


<210>  50
<211>  16
<212>  PRT
<213>  Artificial sequence

<220>
<223>  CDR2-VH

<400>  50

Tyr Ile Ser Tyr Thr Gly Ser Thr Tyr Tyr Asn Pro Ser Leu Lys Ser
1               5                   10                  15


<210>  51
<211>  10
<212>  PRT
<213>  Artificial sequence

<220>
<223>  CDR3-VH

<400>  51

Ala Gly Gly Trp Leu Leu Pro Phe Ala Val
1               5                   10


<210>  52
<211>  17
<212>  PRT
<213>  Artificial sequence

<220>
<223>  CDR1-VL

<400>  52

Lys Ser Ser Gln Ser Leu Leu Tyr Ser Ser Asn Gln Gln Asn Ser Leu
1               5                   10                  15


Ala




<210>  53
<211>  7
<212>  PRT
<213>  Artificial sequence

<220>
<223>  CDR2-VL

<400>  53

Trp Ala Ser Thr Arg Glu Ser
1               5


<210>  54
<211>  9
<212>  PRT
<213>  Artificial sequence

<220>
<223>  CDR3-VL

<400>  54

Gln Gln Tyr Tyr Asn Tyr Pro Trp Thr
1               5


<210>  55
<211>  5
<212>  PRT
<213>  Artificial sequence

<220>
<223>  CDR1-VH

<400>  55

Asp Tyr Gly Met His
1               5


<210>  56
<211>  17
<212>  PRT
<213>  Artificial sequence

<220>
<223>  CDR2-VH

<400>  56

Tyr Ile Ser Ser Gly Ser Ser Thr Ile Tyr Tyr Ala Asp Thr Val Lys
1               5                   10                  15


Gly


<210>  57
<211>  9
<212>  PRT
<213>  Artificial sequence

<220>
<223>  CDR3-VH

<400>  57

Arg Asn Phe Gly Ser Ser Tyr Asp Tyr
1               5

<210>  58
<211>  11
<212>  PRT
<213>  Artificial sequence

<220>
<223>  CDR1-VL

<400>  58

Lys Ala Ser Gln Asp Val Asp Thr Pro Val Ala
1               5                   10

<210>  59
<211>  7
<212>  PRT
<213>  Artificial sequence

<220>
<223>  CDR2-VL

<400>  59

Trp Ala Ser Ile Arg His Thr
1               5

<210>  60
<211>  9
<212>  PRT
<213>  Artificial sequence

<220>
<223>  CDR3-VL

<400>  60

Gln Gln Tyr Ser Ser Tyr Pro Leu Thr
1               5

<210>  61
<211>  5
<212>  PRT
<213>  Artificial sequence

<220>
<223>  CDR1-VH

<400>  61

Ser Asp Tyr Trp Asn
1               5

<210>  62

<211>  16
<212>  PRT
<213>  Artificial sequence

<220>
<223>  CDR2-VH

<400>  62

Tyr Ile Ser Tyr Thr Gly Ser Thr Tyr Tyr Asn Pro Ser Leu Lys Ser
1               5                   10                  15


<210>  63
<211>  10
<212>  PRT
<213>  Artificial sequence

<220>
<223>  CDR3-VH

<400>  63

Ala Gly Gly Trp Leu Leu Pro Phe Ala Val
1               5                   10


<210>  64
<211>  17
<212>  PRT
<213>  Artificial sequence

<220>
<223>  CDR1-VL

<400>  64

Lys Ser Ser Gln Ser Leu Leu Tyr Ser Ser Asn Gln Gln Asn Ser Leu
1               5                   10                  15


Ala




<210>  65
<211>  7
<212>  PRT
<213>  Artificial sequence

<220>
<223>  CDR2-VL

<400>  65

Trp Ala Ser Thr Arg Glu Ser
1               5


<210>  66
<211>  9
<212>  PRT
<213>  Artificial sequence

<220>
<223>  CDR3-VL

<400>  66

Gln Gln Tyr Tyr Ser Tyr Pro Trp Thr
1               5

<210>  67
<211>  5
<212>  PRT
<213>  Artificial sequence

<220>
<223>  CDR1-VH

<400>  67

Ser Gly Tyr Trp Asn
1               5

<210>  68
<211>  16
<212>  PRT
<213>  Artificial sequence

<220>
<223>  CDR2-VH

<400>  68

Tyr Ile Ser Tyr Ser Gly Ser Thr Tyr Tyr Asn Pro Phe Leu Lys Ser
1               5                   10                  15

<210>  69
<211>  10
<212>  PRT
<213>  Artificial sequence

<220>
<223>  CDR3-VH

<400>  69

Met Gly Asp Trp Leu Ala Trp Phe Ala Tyr
1               5                   10

<210>  70
<211>  17
<212>  PRT
<213>  Artificial sequence

<220>
<223>  CDR1-VL

<400>  70

Lys Ser Ser Gln Ser Leu Leu Tyr Ser Ser Asn Gln Lys Asn Ser Leu

1               5                   10                  15

Ala

<210>  71
<211>  7
<212>  PRT
<213>  Artificial sequence

<220>
<223>  CDR2-VL

<400>  71

Trp Ala Ser Thr Arg Glu Ser
1               5

<210>  72
<211>  9
<212>  PRT
<213>  Artificial sequence

<220>
<223>  CDR3-VL

<400>  72

Gln Gln Tyr Tyr Gly Tyr Pro Tyr Thr
1               5

<210>  73
<211>  8
<212>  PRT
<213>  Artificial sequence

<220>
<223>  CDR1-VH

<400>  73

Gly Phe Thr Phe Asn Thr Tyr Gly
1               5

<210>  74
<211>  8
<212>  PRT
<213>  Artificial sequence

<220>
<223>  CDR2-VH

<400>  74

Leu Ser Ser Gly Gly Ser Tyr Thr
1               5

<210>  75
<211>  11
<212>  PRT
<213>  Artificial sequence

<220>
<223>  CDR3-VH

<400>  75

Ala Arg Pro Thr Ala Asp Trp His Leu Asp Val
1               5                   10


<210>  76
<211>  6
<212>  PRT
<213>  Artificial sequence

<220>
<223>  CDR1-VL

<400>  76

Gln Asp Ile Asn Lys Tyr
1               5


<210>  77
<211>  3
<212>  PRT
<213>  Artificial sequence

<220>
<223>  CDR2-VL

<400>  77

Tyr Thr Ser
1


<210>  78
<211>  9
<212>  PRT
<213>  Artificial sequence

<220>
<223>  CDR3-VL

<400>  78

Leu Gln His Asp Asn Leu Leu Phe Thr
1               5


<210>  79
<211>  8
<212>  PRT
<213>  Artificial sequence

<220>
<223>  CDR1-VH

<400>  79

Gly Phe Thr Phe Asn Thr Tyr Gly
1               5

<210>  80
<211>  8
<212>  PRT
<213>  Artificial sequence

<220>
<223>  CDR2-VH

<400>  80

Leu Ser Ser Gly Gly Ser Tyr Thr
1               5

<210>  81
<211>  11
<212>  PRT
<213>  Artificial sequence

<220>
<223>  CDR3-VH

<400>  81

Ala Arg Pro Thr Ala Asp Trp His Leu Asp Val
1               5                   10

<210>  82
<211>  6
<212>  PRT
<213>  Artificial sequence

<220>
<223>  CDR1-VL

<400>  82

Gln Asp Ile Asn Lys Tyr
1               5

<210>  83
<211>  3
<212>  PRT
<213>  Artificial sequence

<220>
<223>  CDR2-VL

<400>  83

Tyr Thr Ser
1

<210>  84
<211>  9
<212>  PRT
<213>  Artificial sequence

<220>
<223>  CDR3-VL

<400>  84

Leu Gln His Asp Asn Leu Leu Phe Thr
1               5


<210>  85
<211>  8
<212>  PRT
<213>  Artificial sequence

<220>
<223>  CDR1-VH

<400>  85

Gly Phe Thr Phe Ser Thr Tyr Gly
1               5


<210>  86
<211>  8
<212>  PRT
<213>  Artificial sequence

<220>
<223>  CDR2-VH

<400>  86

Val Ser Ser Gly Gly Ser Tyr Thr
1               5


<210>  87
<211>  11
<212>  PRT
<213>  Artificial sequence

<220>
<223>  CDR3-VH

<400>  87

Ala Arg Pro Thr Ala Asp Trp His Leu Asp Val
1               5                   10


<210>  88
<211>  6
<212>  PRT
<213>  Artificial sequence

<220>
<223>  CDR1-VL

<400>  88

Gln Asp Ile Asn Arg Tyr
1               5



<210>  89
<211>  3
<212>  PRT
<213>  Artificial sequence

<220>
<223>  CDR2-VL

<400>  89

Tyr Thr Ser
1



<210>  90
<211>  9
<212>  PRT
<213>  Artificial sequence

<220>
<223>  CDR3-VL

<400>  90

Leu Gln His Asp Asn Leu Leu Phe Thr
1               5



<210>  91
<211>  8
<212>  PRT
<213>  Artificial sequence

<220>
<223>  CDR1-VH

<400>  91

Gly Tyr Ser Ile Thr Ser Asp Tyr
1               5



<210>  92
<211>  7
<212>  PRT
<213>  Artificial sequence

<220>
<223>  CDR2-VH

<400>  92

Ile Ser Tyr Thr Gly Ser Thr
1               5

<210>  93
<211>  12
<212>  PRT
<213>  Artificial sequence

<220>
<223>  CDR3-VH

<400>  93

Ala Arg Cys Pro Gly Trp Leu Asn Ala Met Asp Tyr
1               5                   10


<210>  94
<211>  12
<212>  PRT
<213>  Artificial sequence

<220>
<223>  CDR1-VL

<400>  94

Gln Ser Leu Leu Tyr Ser Asn Asn Gln Lys Asn Ser
1               5                   10


<210>  95
<211>  3
<212>  PRT
<213>  Artificial sequence

<220>
<223>  CDR2-VL

<400>  95

Trp Ala Ser
1


<210>  96
<211>  9
<212>  PRT
<213>  Artificial sequence

<220>
<223>  CDR3-VL

<400>  96

Gln Gln Tyr Tyr Gly Tyr Pro Phe Thr
1               5


<210>  97
<211>  8
<212>  PRT
<213>  Artificial sequence

<220>
<223>  CDR1-VH

<400>  97

Gly Tyr Thr Phe Thr Asp Tyr Tyr
1               5

<210>  98
<211>  8
<212>  PRT
<213>  Artificial sequence

<220>
<223>  CDR2-VH

<400>  98

Ile Asn Pro Asn Asn Gly Asp Thr
1               5

<210>  99
<211>  7
<212>  PRT
<213>  Artificial sequence

<220>
<223>  CDR3-VH

<400>  99

Ala Ser Ser Val Met Asp Tyr
1               5

<210>  100
<211>  5
<212>  PRT
<213>  Artificial sequence

<220>
<223>  CDR1-VL

<400>  100

Ser Ser Val Asn Tyr
1               5

<210>  101
<211>  3
<212>  PRT
<213>  Artificial sequence

<220>
<223>  CDR2-VL

<400>  101

Leu Thr Phe
1

<210>  102
<211>  9
<212>  PRT
<213>  Artificial sequence

<220>
<223>  CDR3-VL

<400>  102

Gln Gln Trp Ser Ser Asn Pro Leu Thr
1               5


<210>  103
<211>  8
<212>  PRT
<213>  Artificial sequence

<220>
<223>  CDR1-VH

<400>  103

Gly Tyr Ser Ile Thr Ser Asp Tyr
1               5


<210>  104
<211>  7
<212>  PRT
<213>  Artificial sequence

<220>
<223>  CDR2-VH

<400>  104

Ile Ser Tyr Thr Gly Ser Thr
1               5


<210>  105
<211>  12
<212>  PRT
<213>  Artificial sequence

<220>
<223>  CDR3-VH

<400>  105

Ala Arg Ala Gly Gly Trp Leu Leu Pro Phe Ala Val
1               5                   10


<210>  106
<211>  12
<212>  PRT
<213>  Artificial sequence

<220>
<223>  CDR1-VL

<400>  106

Gln Ser Leu Leu Tyr Ser Ser Asn Gln Gln Asn Ser
1               5                   10


<210>  107
<211>  3
<212>  PRT
<213>  Artificial sequence

<220>
<223>  CDR2-VL

<400>  107

Trp Ala Ser
1


<210>  108
<211>  9
<212>  PRT
<213>  Artificial sequence

<220>
<223>  CDR3-VL

<400>  108

Gln Gln Tyr Tyr Asn Tyr Pro Trp Thr
1               5


<210>  109
<211>  8
<212>  PRT
<213>  Artificial sequence

<220>
<223>  CDR1-VH

<400>  109

Gly Phe Thr Phe Ser Asp Tyr Gly
1               5


<210>  110
<211>  8
<212>  PRT
<213>  Artificial sequence

<220>
<223>  CDR2-VH

<400>  110

Ile Ser Ser Gly Ser Ser Thr Ile
1               5

<210>  111
<211>  11
<212>  PRT
<213>  Artificial sequence

<220>
<223>  CDR3-VH

<400>  111

Ala Arg Arg Asn Phe Gly Ser Ser Tyr Asp Tyr
1               5                   10


<210>  112
<211>  6
<212>  PRT
<213>  Artificial sequence

<220>
<223>  CDR1-VL

<400>  112

Gln Asp Val Asp Thr Pro
1               5


<210>  113
<211>  3
<212>  PRT
<213>  Artificial sequence

<220>
<223>  CDR2-VL

<400>  113

Trp Ala Ser
1


<210>  114
<211>  9
<212>  PRT
<213>  Artificial sequence

<220>
<223>  CDR3-VL

<400>  114

Gln Gln Tyr Ser Ser Tyr Pro Leu Thr
1               5


<210>  115
<211>  8
<212>  PRT
<213>  Artificial sequence

<220>
<223>  CDR1-VH

<400>  115

Gly Tyr Ser Ile Thr Ser Asp Tyr
1               5

<210>  116
<211>  7
<212>  PRT
<213>  Artificial sequence

<220>
<223>  CDR2-VH

<400>  116

Ile Ser Tyr Thr Gly Ser Thr
1               5

<210>  117
<211>  12
<212>  PRT
<213>  Artificial sequence

<220>
<223>  CDR3-VH

<400>  117

Ala Arg Ala Gly Gly Trp Leu Leu Pro Phe Ala Val
1               5                   10

<210>  118
<211>  12
<212>  PRT
<213>  Artificial sequence

<220>
<223>  CDR1-VL

<400>  118

Gln Ser Leu Leu Tyr Ser Ser Asn Gln Gln Asn Ser
1               5                   10

<210>  119
<211>  3
<212>  PRT
<213>  Artificial sequence

<220>
<223>  CDR2-VL

<400>  119

Trp Ala Ser
1

<210>  120
<211>  9
<212>  PRT
<213>  Artificial sequence

<220>
<223>  CDR3-VL

<400>  120

Gln Gln Tyr Tyr Ser Tyr Pro Trp Thr
1               5


<210>  121
<211>  8
<212>  PRT
<213>  Artificial sequence

<220>
<223>  CDR1-VH

<400>  121

Gly Asp Ser Ile Thr Ser Gly Tyr
1               5


<210>  122
<211>  7
<212>  PRT
<213>  Artificial sequence

<220>
<223>  CDR2-VH

<400>  122

Ile Ser Tyr Ser Gly Ser Thr
1               5


<210>  123
<211>  12
<212>  PRT
<213>  Artificial sequence

<220>
<223>  CDR3-VH

<400>  123

Ala Lys Met Gly Asp Trp Leu Ala Trp Phe Ala Tyr
1               5                   10


<210>  124
<211>  12
<212>  PRT
<213>  Artificial sequence

<220>
<223>  CDR1-VL

<400>  124

Gln Ser Leu Leu Tyr Ser Ser Asn Gln Lys Asn Ser
1               5                   10



<210>  125
<211>  3
<212>  PRT
<213>  Artificial sequence

<220>
<223>  CDR2-VL

<400>  125

Trp Ala Ser
1



<210>  126
<211>  9
<212>  PRT
<213>  Artificial sequence

<220>
<223>  CDR3-VL

<400>  126

Gln Gln Tyr Tyr Gly Tyr Pro Tyr Thr
1               5



<210>  127
<211>  114
<212>  PRT
<213>  Artificial sequence

<220>
<223>  Heavy chain variable region

<400>  127

Gln Val Gln Leu Val Gln Ser Gly Pro Glu Leu Lys Lys Pro Gly Ala
1               5                   10                  15


Ser Val Lys Ile Ser Cys Lys Ala Ser Gly Tyr Thr Phe Thr Asp Tyr
            20                  25                  30


Tyr Met Asn Trp Val Arg Gln Ala Pro Gly Gln Ser Leu Glu Trp Ile
        35                  40                  45


Gly Asp Ile Trp Pro Asn Asn Gly Asp Thr Trp Tyr Asn Gln Lys Phe
    50                  55                  60


Lys Gly Arg Val Thr Leu Thr Arg Asp Thr Ser Thr Ser Thr Val Tyr
65                  70                  75                  80

Met Glu Leu Arg Ser Leu Arg Ser Glu Asp Thr Ala Val Tyr Tyr Cys
                85                  90                  95

Ala Arg Ser Val Met Asp Tyr Trp Gly Gln Gly Thr Leu Val Thr Val
            100                 105                 110

Ser Ser

<210>  128
<211>  106
<212>  PRT
<213>  Artificial sequence

<220>
<223>  Light chain variable region

<400>  128

Glu Ile Val Leu Thr Gln Ser Pro Ala Leu Leu Ser Leu Ser Pro Gly
1               5                   10                  15

Glu Arg Val Thr Leu Ser Cys Ser Ala Ser Ser Ser Val Asn Tyr Val
            20                  25                  30

Tyr Trp Tyr Gln Gln Lys Pro Gly Gln Ala Pro Arg Pro Leu Ile Tyr
        35                  40                  45

Leu Thr Phe Asn Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Val Asn Pro Leu Thr
                85                  90                  95

Phe Gly Gly Gly Thr Lys Val Glu Ile Lys
            100                 105

<210>  129
<211>  114
<212>  PRT
<213>  Artificial sequence

<220>
<223>  Heavy chain variable region

<400>  129

Gln Val Gln Leu Val Gln Ser Gly Pro Glu Leu Lys Lys Pro Gly Ala

1               5                   10                  15

Ser Val Lys Ile Ser Cys Lys Ala Ser Gly Tyr Thr Phe Thr Asp Tyr
            20                  25                  30

Tyr Met Asn Trp Val Arg Gln Ala Pro Gly Gln Ser Leu Glu Trp Ile
        35                  40                  45

Gly Asp Ile Trp Pro Asn Asn Gly Asp Thr Ser Tyr Asn Gln Lys Phe
    50                  55                  60

Lys Gly Arg Val Thr Leu Thr Arg Asp Thr Ser Thr Ser Thr Val Tyr
65                  70                  75                  80

Met Glu Leu Arg Ser Leu Arg Ser Glu Asp Thr Ala Val Tyr Tyr Cys
                85                  90                  95

Ala Arg Ser Val Met Asp Thr Trp Gly Gln Gly Thr Leu Val Thr Val
            100                 105                 110

Ser Ser

<210>  130
<211>  106
<212>  PRT
<213>  Artificial sequence

<220>
<223>  Light chain variable region

<400>  130

Glu Ile Val Leu Thr Gln Ser Pro Ala Leu Leu Ser Leu Ser Pro Gly
1               5                   10                  15

Glu Arg Val Thr Leu Ser Cys Ser Ala Ser Ser Ser Val Asn Tyr Val
            20                  25                  30

Tyr Trp Tyr Gln Gln Lys Pro Gly Gln Ala Pro Arg Pro Leu Ile Tyr
        35                  40                  45

Leu Thr Phe Asn Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Val Asn Pro Leu Thr
                85                  90                  95

Phe Gly Gly Gly Thr Lys Val Glu Ile Lys
            100                 105

<210>  131
<211>  114
<212>  PRT
<213>  Artificial sequence

<220>
<223>  Heavy chain variable region

<400>  131

Gln Val Gln Leu Val Gln Ser Gly Pro Glu Leu Lys Lys Pro Gly Ala
1               5                   10                  15

Ser Val Lys Ile Ser Cys Lys Ala Ser Gly Tyr Thr Phe Thr Asp Tyr
            20                  25                  30

Tyr Met Asn Trp Val Arg Gln Ala Pro Gly Gln Ser Leu Glu Trp Ile
        35                  40                  45

Gly Asp Ile Trp Pro Asn Asn Gly Asp Thr Ser Tyr Asn Gln Lys Phe
    50                  55                  60

Lys Gly Arg Val Thr Leu Thr Arg Asp Thr Ser Thr Ser Thr Val Tyr
65                  70                  75                  80

Met Glu Leu Arg Ser Leu Arg Ser Glu Asp Thr Ala Val Tyr Tyr Cys
                85                  90                  95

Ala Arg Ser Val Met Ser Asp Trp Gly Gln Gly Thr Leu Val Thr Val
            100                 105                 110

Ser Ser

<210>  132
<211>  106
<212>  PRT
<213>  Artificial sequence

<220>
<223>  Light chain variable region

<400>  132

Glu Ile Val Leu Thr Gln Ser Pro Ala Leu Leu Ser Leu Ser Pro Gly
1               5                   10                  15

Glu Arg Val Thr Leu Ser Cys Ser Ala Ser Ser Ser Val Asn Tyr Val

20                  25                  30

Tyr Trp Tyr Gln Gln Lys Pro Gly Gln Ala Pro Arg Pro Leu Ile Tyr
        35                  40                  45

Leu Thr Phe Asn Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Leu Thr
                85                  90                  95

Phe Gly Gly Gly Thr Lys Val Glu Ile Lys
            100                 105

<210>  133
<211>  114
<212>  PRT
<213>  Artificial sequence

<220>
<223>  Heavy chain variable region

<400>  133

Gln Val Gln Leu Val Gln Ser Gly Pro Glu Leu Lys Lys Pro Gly Ala
1               5                   10                  15

Ser Val Lys Ile Ser Cys Lys Ala Ser Gly Tyr Thr Phe Thr Asp Tyr
            20                  25                  30

Tyr Met Asn Trp Val Arg Gln Ala Pro Gly Gln Ser Leu Glu Trp Ile
        35                  40                  45

Gly Asp Ile Trp Pro Asn Asn Gly Asp Thr Ser Tyr Asn Gln Lys Phe
    50                  55                  60

Lys Gly Arg Val Thr Leu Thr Arg Asp Thr Ser Thr Ser Thr Val Tyr
65                  70                  75                  80

Met Glu Leu Arg Ser Leu Arg Ser Glu Asp Thr Ala Val Tyr Tyr Cys
                85                  90                  95

Ala Arg Ser Val Met Asp Tyr Trp Gly Gln Gly Thr Leu Val Thr Val
            100                 105                 110

Ser Ser

<210>  134
<211>  106
<212>  PRT
<213>  Artificial sequence

<220>
<223>  Light chain variable region

<400>  134

Glu Ile Val Leu Thr Gln Ser Pro Ala Leu Leu Ser Leu Ser Pro Gly
1               5                   10                  15

Glu Arg Val Thr Leu Ser Cys Ser Ala Ser Ser Ser Val Asn Tyr Val
            20                  25                  30

Tyr Trp Tyr Gln Gln Lys Pro Gly Gln Ala Pro Arg Pro Leu Ile Tyr
        35                  40                  45

Leu Thr Phe Asn Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Val Asn Pro Leu Thr
                85                  90                  95

Phe Gly Gly Gly Thr Lys Val Glu Ile Lys
            100                 105


<210>  135
<211>  5
<212>  PRT
<213>  Artificial sequence

<220>
<223>  CDR1-VH

<400>  135

Asp Tyr Tyr Met Asn
1               5


<210>  136
<211>  17
<212>  PRT
<213>  Artificial sequence

<220>
<223>  CDR2-VH

<400>  136

Asp Ile Trp Pro Asn Asn Gly Asp Thr Trp Tyr Asn Gln Lys Phe Lys
1               5                   10                  15

Gly

<210>  137
<211>  5
<212>  PRT
<213>  Artificial sequence

<220>
<223>  CDR3-VH

<400>  137

Ser Val Met Asp Tyr
1               5

<210>  138
<211>  10
<212>  PRT
<213>  Artificial sequence

<220>
<223>  CDR1-VL

<400>  138

Ser Ala Ser Ser Ser Val Asn Tyr Val Tyr
1               5                   10

<210>  139
<211>  7
<212>  PRT
<213>  Artificial sequence

<220>
<223>  CDR2-VL

<400>  139

Leu Thr Phe Asn Leu Ala Ser
1               5

<210>  140
<211>  9
<212>  PRT
<213>  Artificial sequence

<220>
<223>  CDR3-VL

<400>  140

Gln Gln Trp Ser Val Asn Pro Leu Thr
1               5

<210>  141
<211>  5
<212>  PRT
<213>  Artificial sequence

<220>
<223>  CDR1-VH

<400>  141

Asp Tyr Tyr Met Asn
1               5


<210>  142
<211>  17
<212>  PRT
<213>  Artificial sequence

<220>
<223>  CDR2-VH

<400>  142

Asp Ile Trp Pro Asn Asn Gly Asp Thr Ser Tyr Asn Gln Lys Phe Lys
1               5                   10                  15


Gly



<210>  143
<211>  5
<212>  PRT
<213>  Artificial sequence

<220>
<223>  CDR3-VH

<400>  143

Ser Val Met Asp Thr
1               5


<210>  144
<211>  10
<212>  PRT
<213>  Artificial sequence

<220>
<223>  CDR1-VL

<400>  144

Ser Ala Ser Ser Ser Val Asn Tyr Val Tyr
1               5                   10


<210>  145

<211>  7
<212>  PRT
<213>  Artificial sequence

<220>
<223>  CDR2-VL

<400>  145

Leu Thr Phe Asn Leu Ala Ser
1               5


<210>  146
<211>  9
<212>  PRT
<213>  Artificial sequence

<220>
<223>  CDR3-VL

<400>  146

Gln Gln Trp Ser Val Asn Pro Leu Thr
1               5


<210>  147
<211>  5
<212>  PRT
<213>  Artificial sequence

<220>
<223>  CDR1-VH

<400>  147

Asp Tyr Tyr Met Asn
1               5


<210>  148
<211>  17
<212>  PRT
<213>  Artificial sequence

<220>
<223>  CDR2-VH

<400>  148

Asp Ile Trp Pro Asn Asn Gly Asp Thr Ser Tyr Asn Gln Lys Phe Lys
1               5                   10                  15


Gly


<210>  149
<211>  5
<212>  PRT
<213>  Artificial sequence

<220>
<223>  CDR3-VH

<400>  149

Ser Val Met Ser Asp
1               5


<210>  150
<211>  10
<212>  PRT
<213>  Artificial sequence

<220>
<223>  CDR1-VL

<400>  150

Ser Ala Ser Ser Ser Val Asn Tyr Val Tyr
1               5                   10


<210>  151
<211>  7
<212>  PRT
<213>  Artificial sequence

<220>
<223>  CDR2-VL

<400>  151

Leu Thr Phe Asn Leu Ala Ser
1               5


<210>  152
<211>  9
<212>  PRT
<213>  Artificial sequence

<220>
<223>  CDR3-VL

<400>  152

Gln Gln Trp Ser Ser Asn Pro Leu Thr
1               5


<210>  153
<211>  5
<212>  PRT
<213>  Artificial sequence

<220>
<223>  CDR1-VH

<400>  153

Asp Tyr Tyr Met Asn

1                 5


<210>  154
<211>  17
<212>  PRT
<213>  Artificial sequence

<220>
<223>  CDR2-VH

<400>  154

Asp Ile Trp Pro Asn Asn Gly Asp Thr Ser Tyr Asn Gln Lys Phe Lys
1               5                   10                  15


Gly




<210>  155
<211>  5
<212>  PRT
<213>  Artificial sequence

<220>
<223>  CDR3-VH

<400>  155

Ser Val Met Asp Tyr
1               5


<210>  156
<211>  10
<212>  PRT
<213>  Artificial sequence

<220>
<223>  CDR1-VL

<400>  156

Ser Ala Ser Ser Ser Val Asn Tyr Val Tyr
1               5                   10


<210>  157
<211>  7
<212>  PRT
<213>  Artificial sequence

<220>
<223>  CDR2-VL

<400>  157

Leu Thr Phe Asn Leu Ala Ser
1               5

<210>  158
<211>  9
<212>  PRT
<213>  Artificial sequence

<220>
<223>  CDR3-VL

<400>  158

Gln Gln Trp Ser Val Asn Pro Leu Thr
1               5


<210>  159
<211>  8
<212>  PRT
<213>  Artificial sequence

<220>
<223>  CDR1-VH

<400>  159

Gly Tyr Thr Phe Thr Asp Tyr Tyr
1               5


<210>  160
<211>  8
<212>  PRT
<213>  Artificial sequence

<220>
<223>  CDR2-VH

<400>  160

Ile Trp Pro Asn Asn Gly Asp Thr
1               5


<210>  161
<211>  7
<212>  PRT
<213>  Artificial sequence

<220>
<223>  CDR3-VH

<400>  161

Ala Arg Ser Val Met Asp Tyr
1               5


<210>  162
<211>  5
<212>  PRT
<213>  Artificial sequence

<220>
<223>  CDR1-VL

<400>  162

Ser Ser Val Asn Tyr
1               5



<210>  163
<211>  3
<212>  PRT
<213>  Artificial sequence

<220>
<223>  CDR2-VL

<400>  163

Leu Thr Phe
1



<210>  164
<211>  9
<212>  PRT
<213>  Artificial sequence

<220>
<223>  CDR3-VL

<400>  164

Gln Gln Trp Ser Val Asn Pro Leu Thr
1               5



<210>  165
<211>  8
<212>  PRT
<213>  Artificial sequence

<220>
<223>  CDR1-VH

<400>  165

Gly Tyr Thr Phe Thr Asp Tyr Tyr
1               5



<210>  166
<211>  8
<212>  PRT
<213>  Artificial sequence

<220>
<223>  CDR2-VH

<400>  166

Ile Trp Pro Asn Asn Gly Asp Thr
1               5

<210>  167
<211>  7
<212>  PRT
<213>  Artificial sequence

<220>
<223>  CDR3-VH

<400>  167

Ala Arg Ser Val Met Asp Thr
1               5


<210>  168
<211>  5
<212>  PRT
<213>  Artificial sequence

<220>
<223>  CDR1-VL

<400>  168

Ser Ser Val Asn Tyr
1               5


<210>  169
<211>  3
<212>  PRT
<213>  Artificial sequence

<220>
<223>  CDR2-VL

<400>  169

Leu Thr Phe
1


<210>  170
<211>  9
<212>  PRT
<213>  Artificial sequence

<220>
<223>  CDR3-VL

<400>  170

Gln Gln Trp Ser Val Asn Pro Leu Thr
1               5


<210>  171
<211>  8
<212>  PRT
<213>  Artificial sequence

<220>
<223>  CDR1-VH

<400>  171

Gly Tyr Thr Phe Thr Asp Tyr Tyr
1               5


<210>  172
<211>  8
<212>  PRT
<213>  Artificial sequence

<220>
<223>  CDR2-VH

<400>  172

Ile Trp Pro Asn Asn Gly Asp Thr
1               5


<210>  173
<211>  7
<212>  PRT
<213>  Artificial sequence

<220>
<223>  CDR3-VH

<400>  173

Ala Arg Ser Val Met Ser Asp
1               5


<210>  174
<211>  5
<212>  PRT
<213>  Artificial sequence

<220>
<223>  CDR1-VL

<400>  174

Ser Ser Val Asn Tyr
1               5


<210>  175
<211>  3
<212>  PRT
<213>  Artificial sequence

<220>
<223>  CDR2-VL

<400>  175

Leu Thr Phe
1

<210>  176
<211>  9
<212>  PRT
<213>  Artificial sequence

<220>
<223>  CDR3-VL

<400>  176

Gln Gln Trp Ser Ser Asn Pro Leu Thr
1               5


<210>  177
<211>  8
<212>  PRT
<213>  Artificial sequence

<220>
<223>  CDR1-VH

<400>  177

Gly Tyr Thr Phe Thr Asp Tyr Tyr
1               5


<210>  178
<211>  8
<212>  PRT
<213>  Artificial sequence

<220>
<223>  CDR2-VH

<400>  178

Ile Trp Pro Asn Asn Gly Asp Thr
1               5


<210>  179
<211>  7
<212>  PRT
<213>  Artificial sequence

<220>
<223>  CDR3-VH

<400>  179

Ala Arg Ser Val Met Asp Tyr
1               5


<210>  180
<211>  5
<212>  PRT
<213>  Artificial sequence

<220>
<223>  CDR1-VL

<400>  180

Ser Ser Val Asn Tyr
1               5



<210>  181
<211>  3
<212>  PRT
<213>  Artificial sequence

<220>
<223>  CDR2-VL

<400>  181

Leu Thr Phe
1



<210>  182
<211>  9
<212>  PRT
<213>  Artificial sequence

<220>
<223>  CDR3-VL

<400>  182

Gln Gln Trp Ser Val Asn Pro Leu Thr
1               5

## Description

This application claims the priority of Chinese Patent Application No. 201911088643.5, filed with the China National Intellectual Property Administration on November 08, 2019, and titled with "ANTI-HUMAN PROGRAMMED CELL DEATH LIGAND-1 (PD-L1) ANTIBODY AND USE THEREOF", which is hereby incorporated by reference in entirety.

### FIELD

The present invention relates to antibodies against human programmed death ligand-1 (PD-L1) or antigen-binding fragments thereof, nucleic acids encoding the same, their expression vectors and expression cells, preparation methods, pharmaceutical compositions, and their use for enhancing the function of T cells, upregulating T cell-mediated immune responses and for the treatment of diseases related to abnormal expression of PD-L1 and abnormal function of T cells, such as tumors.

### BACKGROUND

Immunotherapy has become one of the most rapidly developing and promising research fields in tumor therapy. The use of immune checkpoint inhibitors, such as PD-1/PD-L1 monoclonal antibody and CTLA-4 monoclonal antibody, is a revolutionary treatment method for tumor immunotherapy, which has greatly improved the survival time of patients with malignant tumor.

The immune response mediated by T cells is strictly regulated by co-stimulation and co-inhibition mechanisms and maintains an optimal balance between antigen immune response and maintenance of self-tolerance. This balance is engaged by a variety of activating and inhibitory proteins. Inhibitory proteins, also known as immune checkpoint proteins, regulate the activation and effector function of cytotoxic T lymphocytes (CTLs) to maintain self-tolerance. Immune checkpoint inhibitor proteins play a key role in tumor regulatory pathways. One of the important immune checkpoint proteins, PD-1, upon binding to its ligand PD-L1, can transmit immunosuppressive signals and reduce the activity of T cells. Tumor cells can also express PD-L1 on the cell surface to inhibit the activation and proliferation of T cells , thus escaping from the attack and killing by CTLs. Using PD-1 or PD-L1 monoclonal antibody to prevent the binding and interaction of PD-1/PD-L1 can partially restore the function of T cells, thus enhancing the ability to kill tumor cells.

In 2011, ipilimumab, an anti-CTLA-4 monoclonal antibody and the first immune check point inhibitor, became a successful tumor immunotherapy for the treatment of melanoma. Up to now, many patients treated by this immunotherapy have obtained a longer 5-year survival time than traditional treatment methods. Since then, FDA has successively approved 3 PD-1 monoclonal antibodies and 3 PD-L1 monoclonal antibodies, which have been successfully used in immunotherapy for more than a dozen tumors in addition to melanoma and have become the first-line treatment for various cancers, such as non-small cell lung cancer (NSCLC), renal cell carcinoma (RCC) and bladder or urothelial carcinoma. China has so far approved the listing of 2 imported PD-1 antibodies and 3 domestic PD-1 antibodies, but no PD-L1 antibody has been approved. In view of the differences in the therapeutic mechanism between PD-L1 antibody and PD-1 antibody, as well as in the combination drugs for current clinical trials and in applicable indications, the development of new PD-L1 monoclonal antibodies and PD-L1-based double antibodies still has great social and economic significance.

### SUMMARY

The present invention provides an antibody and antigen-binding fragment thereof that specifically binds to human programmed death ligand-1 (PD-L1), a nucleic acid encoding the antibody and antigen-binding fragment thereof, a pharmaceutical composition and kit comprising the antibody and antigen-binding fragment thereof, and use for enhancing T cell function, upregulating T cell-mediated immune responses and for the treatment of a disease associated with abnormal PD-L1 expression and abnormal T cell function, such as tumor. The antibody can not only bind to human and cynomolgus monkey PD-L1 protein, but also block the interaction between human PD-L1 and human PD-1.

In some embodiments, an isolated antibody or antigen-binding fragment thereof specifically binding to human programmed death ligand-1 (PD-L1) is provided, comprising a combination of heavy chain CDRs and a combination of light chain CDRs:
(1) the combination of heavy chain CDRs comprises CDR1-VH, CDR2-VH and CDR3-VH; the CDR1-VH, CDR2-VH and CDR3-VH have sequences selected from the group consisting of the following combinations or sequences with insertion, deletion and/or substitution of 1, 2, 3 or more amino acids compared to the following combinations:

| Combination No. | SEQ ID NO: | | |
|---|---|---|---|
| | CDR1-VH | CDR2-VH | CDR3-VH |
| VH1 | SEQ ID NO: 19 | SEQ ID NO: 20 | SEQ ID NO: 21 |
| VH2 | SEQ ID NO: 25 | SEQ ID NO: 26 | SEQ ID NO: 27 |
| VH3 | SEQ ID NO: 31 | SEQ ID NO: 32 | SEQ ID NO: 33 |
| VH4 | SEQ ID NO: 37 | SEQ ID NO: 38 | SEQ ID NO: 39 |
| VH5 | SEQ ID NO: 43 | SEQ ID NO: 44 | SEQ ID NO: 45 |
| VH6 | SEQ ID NO: 49 | SEQ ID NO: 50 | SEQ ID NO: 51 |
| VH7 | SEQ ID NO: 55 | SEQ ID NO: 56 | SEQ ID NO: 57 |
| VH8 | SEQ ID NO: 61 | SEQ ID NO: 62 | SEQ ID NO: 63 |
| VH9 | SEQ ID NO: 67 | SEQ ID NO: 68 | SEQ ID NO: 69 |
| VH10 | SEQ ID NO: 73 | SEQ ID NO: 74 | SEQ ID NO: 75 |
| VH11 | SEQ ID NO: 79 | SEQ ID NO: 80 | SEQ ID NO: 81 |
| VH12 | SEQ ID NO: 85 | SEQ ID NO: 86 | SEQ ID NO: 87 |
| VH13 | SEQ ID NO: 91 | SEQ ID NO: 92 | SEQ ID NO: 93 |
| VH14 | SEQ ID NO: 97 | SEQ ID NO: 98 | SEQ ID NO: 99 |
| VH15 | SEQ ID NO: 103 | SEQ ID NO: 104 | SEQ ID NO: 105 |
| VH16 | SEQ ID NO: 109 | SEQ ID NO: 110 | SEQ ID NO: 111 |
| VH17 | SEQ ID NO: 115 | SEQ ID NO: 116 | SEQ ID NO: 117 |
| VH18 | SEQ ID NO: 121 | SEQ ID NO: 122 | SEQ ID NO: 123 |

and
(2) the combination of light chain CDRs comprises CDR1-VL, CDR2-VL and CDR3-VL; the CDR1-VL, CDR2-VL and CDR3-VL have sequences selected from the group consisting of the following combinations or sequences with insertion, deletion and/or substitution of 1, 2, 3 or more amino acids compared to the following combinations:

| Combination No. | SEQ ID NO: | | |
|---|---|---|---|
| | CDR1-VL | CDR2-VL | CDR3-VL |
| VL1 | SEQ ID NO: 22 | SEQ ID NO: 23 | SEQ ID NO: 24 |
| VL2 | SEQ ID NO: 28 | SEQ ID NO: 29 | SEQ ID NO: 30 |
| VL3 | SEQ ID NO: 34 | SEQ ID NO: 35 | SEQ ID NO: 36 |
| VL4 | SEQ ID NO: 40 | SEQ ID NO: 41 | SEQ ID NO: 42 |
| VL5 | SEQ ID NO: 46 | SEQ ID NO: 47 | SEQ ID NO: 48 |
| VL6 | SEQ ID NO: 52 | SEQ ID NO: 53 | SEQ ID NO: 54 |
| VL7 | SEQ ID NO: 58 | SEQ ID NO: 59 | SEQ ID NO: 60 |
| VL8 | SEQ ID NO: 64 | SEQ ID NO: 65 | SEQ ID NO: 66 |
| VL9 | SEQ ID NO: 70 | SEQ ID NO: 71 | SEQ ID NO: 72 |
| VL10 | SEQ ID NO: 76 | SEQ ID NO: 77 | SEQ ID NO: 78 |
| VL11 | SEQ ID NO: 82 | SEQ ID NO: 83 | SEQ ID NO: 84 |
| VL12 | SEQ ID NO: 88 | SEQ ID NO: 89 | SEQ ID NO: 90 |
| VL13 | SEQ ID NO: 94 | SEQ ID NO: 95 | SEQ ID NO: 96 |
| VL14 | SEQ ID NO: 100 | SEQ ID NO: 101 | SEQ ID NO: 102 |
| VL15 | SEQ ID NO: 106 | SEQ ID NO: 107 | SEQ ID NO: 108 |
| VL16 | SEQ ID NO: 112 | SEQ ID NO: 113 | SEQ ID NO: 114 |
| VL17 | SEQ ID NO: 118 | SEQ ID NO: 119 | SEQ ID NO: 120 |
| VL18 | SEQ ID NO: 124 | SEQ ID NO: 125 | SEQ ID NO: 126 |

each of CDR1-VH, CDR2-VH, CDR3-VH, CDR1-VL, CDR2-VL and CDR3-VL is defined by a common analysis method of KABAT, Chothia or IMGT numbering.

In some embodiments, an isolated humanized antibody or antigen-binding fragment thereof specifically binding to human programmed death ligand-1 (PD-L1) is provided, comprising a combination of heavy chain CDRs and a combination of light chain CDRs:
(1) the combination of heavy chain CDRs comprises CDR1-VH, CDR2-VH and CDR3-VH; the CDR1-VH, CDR2-VH and CDR3-VH have sequences selected from the group consisting of the following combinations or sequences with insertion, deletion and/or substitution of 1, 2, 3 or more amino acids compared to the following combinations:

| Combination No. | SEQ ID NO: | | |
|---|---|---|---|
| | CDR1-VH | CDR2-VH | CDR3-VH |
| VH19 | SEQ ID NO: 135 | SEQ ID NO: 136 | SEQ ID NO: 137 |
| VH20 | SEQ ID NO: 141 | SEQ ID NO: 142 | SEQ ID NO: 143 |
| VH21 | SEQ ID NO: 147 | SEQ ID NO: 148 | SEQ ID NO: 149 |
| VH22 | SEQ ID NO: 153 | SEQ ID NO: 154 | SEQ ID NO: 155 |
| VH23 | SEQ ID NO: 159 | SEQ ID NO: 160 | SEQ ID NO: 161 |
| VH24 | SEQ ID NO: 165 | SEQ ID NO: 166 | SEQ ID NO: 167 |
| VH25 | SEQ ID NO: 171 | SEQ ID NO: 172 | SEQ ID NO: 173 |
| VH26 | SEQ ID NO: 177 | SEQ ID NO: 178 | SEQ ID NO: 179 |

and
(2) the combination of light chain CDRs comprises CDR1-VL, CDR2-VL and CDR3-VL; the CDR1-VL, CDR2-VL and CDR3-VL have sequences selected from the group consisting of the following combinations or sequences with insertion, deletion and/or substitution of 1, 2, 3 or more amino acids compared to the following combinations:

| Combination No. | SEQ ID NO: | | |
|---|---|---|---|
| | CDR1-VL | CDR2-VL | CDR3-VL |
| VL19 | SEQ ID NO: 138 | SEQ ID NO: 139 | SEQ ID NO: 140 |
| VL20 | SEQ ID NO: 144 | SEQ ID NO: 145 | SEQ ID NO: 146 |
| VL21 | SEQ ID NO: 150 | SEQ ID NO: 151 | SEQ ID NO: 152 |
| VL22 | SEQ ID NO: 156 | SEQ ID NO: 157 | SEQ ID NO: 158 |
| VL23 | SEQ ID NO: 162 | SEQ ID NO: 163 | SEQ ID NO: 164 |
| VL24 | SEQ ID NO: 168 | SEQ ID NO: 169 | SEQ ID NO: 170 |
| VL25 | SEQ ID NO: 174 | SEQ ID NO: 175 | SEQ ID NO: 176 |
| VL26 | SEQ ID NO: 180 | SEQ ID NO: 181 | SEQ ID NO: 182 |

each of CDR1-VH, CDR2-VH, CDR3-VH, CDR1-VL, CDR2-VL and CDR3-VL is defined by a common analysis method of KABAT, Chothia or IMGT numbering.

In particular, for example, the antibody or antigen-binding fragment thereof of the present invention comprises a combination of heavy chain CDRs and light chain CDRs, and the combination of heavy chain CDRs and light chain CDRs is selected from the group consisting of VH1+VL1, VH2+VL2, VH3+VL3, VH4+VL4, VH5+VL5, VH6+VL6, VH7+VL7, VH8+VL8, VH9+VL9, VH10+VL10, VH11+VL11, VH12+VL12, VH13+VL13, VH14+VL14, VH15+VL15, VH16+VL16, VH17+VL17, VH18+VL18, VH19+VL19, VH20+VL20, VH21+VL21, VH22+VL22, VH23+VL23, VH24+VL24, VH25+VL25, VH26+VL26 and CDR combinations having sequences with insertion, deletion and/or substitution of 1, 2, 3 or more amino acids compared to the sequences of combination of the heavy chain CDRs and light chain CDRs.

In another specific embodiment, the present invention provides an antibody or antigen-binding fragment thereof comprises:
1) a heavy chain variable region and a light chain variable region having the sequences set forth in SEQ ID NO: 1 and SEQ ID NO: 2, respectively, or sequences having 70%, 75%, 80%, 85%, 90%, 95%, 96%, 97%, 98%, 99% or higher identity to the aforementioned sequences;
2) a heavy chain variable region and a light chain variable region having the sequences set forth in SEQ ID NO: 3 and SEQ ID NO: 4, respectively, or sequences having 70%, 75%, 80%, 85%, 90%, 95%, 96%, 97%, 98%, 99% or higher identity to the aforementioned sequences;
3) a heavy chain variable region and a light chain variable region having the sequences set forth in SEQ ID NO: 5 and SEQ ID NO: 6, respectively, or sequences having 70%, 75%, 80%, 85%, 90%, 95%, 96%, 97%, 98%, 99% or higher identity to the aforementioned sequences;
4) a heavy chain variable region and a light chain variable region having the sequences set forth in SEQ ID NO: 7 and SEQ ID NO: 8, respectively, or sequences having 70%, 75%, 80%, 85%, 90%, 95%, 96%, 97%, 98%, 99% or higher identity to the aforementioned sequences;
5) a heavy chain variable region and a light chain variable region having the sequences set forth in SEQ ID NO: 9 and SEQ ID NO: 10, respectively, or sequences having 70%, 75%, 80%, 85%, 90%, 95%, 96%, 97%, 98%, 99% or higher identity to the aforementioned sequences;
6) a heavy chain variable region and a light chain variable region having the sequences set forth in SEQ ID NO: 11 and SEQ ID NO: 12, respectively, or sequences having 70%, 75%, 80%, 85%, 90%, 95%, 96%, 97%, 98%, 99% or higher identity to the aforementioned sequences;
7) a heavy chain variable region and a light chain variable region having the sequences set forth in SEQ ID NO: 13 and SEQ ID NO: 14, respectively, or sequences having 70%, 75%, 80%, 85%, 90%, 95%, 96%, 97%, 98%, 99% or higher identity to the aforementioned sequences;
8) a heavy chain variable region and a light chain variable region having the sequences set forth in SEQ ID NO: 15 and SEQ ID NO: 16, respectively, or sequences having 70%, 75%, 80%, 85%, 90%, 95%, 96%, 97%, 98%, 99% or higher identity to the aforementioned sequences;
9) a heavy chain variable region and a light chain variable region having the sequences set forth in SEQ ID NO: 17 and SEQ ID NO: 18, respectively, or sequences having 70%, 75%, 80%, 85%, 90%, 95%, 96%, 97%, 98%, 99% or higher identity to the aforementioned sequences;
10) a heavy chain variable region and a light chain variable region having the sequences set forth in SEQ ID NO: 127 and SEQ ID NO: 128, respectively, or sequences having 70%, 75%, 80%, 85%, 90%, 95%, 96%, 97%, 98%, 99% or higher identity to the aforementioned sequences;
11) a heavy chain variable region and a light chain variable region having the sequences set forth in SEQ ID NO: 129 and SEQ ID NO: 130, respectively, or sequences having 70%, 75%, 80%, 85%, 90%, 95%, 96%, 97%, 98%, 99% or higher identity to the aforementioned sequences;
12) a heavy chain variable region and a light chain variable region having the sequences set forth in SEQ ID NO: 131 and SEQ ID NO: 132, respectively, or sequences having 70%, 75%, 80%, 85%, 90%, 95%, 96%, 97%, 98%, 99% or higher identity to the aforementioned sequences; or
13) a heavy chain variable region and a light chain variable region having the sequences set forth in SEQ ID NO: 133 and SEQ ID NO: 134, respectively, or sequences having 70%, 75%, 80%, 85%, 90%, 95%, 96%, 97%, 98%, 99% or higher identity to the aforementioned sequences.

In a preferred embodiment, the antibody or antigen-binding fragment thereof of the present invention is chimeric or humanized or fully humanized.

In a preferred embodiment, the antibody or antigen-binding fragment thereof of the present invention has a dissociation constant (KD) of no more than 10 nM for binding to human programmed death ligand-1 (PD-L1), and a dissociation constant (KD) of no more than 100 nM for binding to cynomolgus monkey programmed death ligand-1 (PD-L1).

In a preferred embodiment, the antibody or antigen-binding fragment thereof of the present invention comprises a constant region selected from the group consisting of human or murine IgG1, IgG2, IgG3, IgG4, IgA, IgM, IgE and IgD; preferably a constant region selected from the group consisting of human or murine IgG1, IgG2, IgG3 and IgG4; or a constant region selected from the group consisting of mutated human or murine IgG1, IgG2, IgG3 or IgG4.

In a preferred embodiment, the antigen-binding fragment thereof of the present invention is selected from the group consisting of F(ab)₂, Fab', Fab, Fv, scFv, bispecific antibody, nanobody, a minimum recognition unit of an antibody and a combination thereof.

In a preferred embodiment, the antibody or the antigen-binding fragment thereof of the present invention can competitively bind to PD-L1 with an antibody selected from the group consisting of antibodies numbered 34, 50, 90, 130, 156, 370, 373, 413 and 794, and has characteristics of:
1) specifically binding to a PD-L1 recombinant protein and a cell expressing PD-L1;
2) blocking the binding of PD-L1 to PD-1 protein;
3) suppressing the binding of PD-1 to PD-L1 expressed on cell surface;
4) enhancing the activity of T cells;
5) mediating antibody-dependent cell-mediated cytotoxicity (ADCC) activity; or/and
6) inhibiting tumor growth.

In some embodiments, the present invention provides an isolated nucleic acid encoding the antibody or antigen-binding fragment thereof, or any combination thereof described above.

In some embodiments, the present invention provides an expression vector comprising the above-described isolated nucleic acid of the present invention.

In some embodiments, the present invention provides a host cell comprising the isolated nucleic acid or expression vector of the present invention described above.

In a preferred embodiment, the host cell is a eukaryotic cell or a prokaryotic cell; more preferably, the host cell is derived from mammalian cells, yeast cells, insect cells, Escherichia coli and/or Bacillus subtilis; more preferably, the host cell is Chinese Hamster Ovary (CHO) cells.

In some embodiments, the present invention provides a method for producing an antibody or antigen-binding fragment thereof, comprising culturing the host cell of the present invention described above under a suitable condition and isolating the antibody or antigen-binding fragment thereof.

In some embodiments, the present invention provides a pharmaceutical composition, which comprises the antibody or antigen-binding fragment thereof of the invention described above, the isolated nucleic acid of the invention described above, the expression vector of the invention described above, the cell of the invention described above, or the product (e.g. antibody and antigen-binding fragment thereof) produced by the method of the invention described above, and a pharmaceutically acceptable carrier.

In a preferred embodiment, the pharmaceutical composition further comprises an additional anti-tumor agent.

In some embodiments, the present invention provides a method for preventing and/or treating a disease associated with abnormal expression of PD-L1 and/or abnormal T cell function, comprising administering to a subject in need thereof the antibody or antigen-binding fragment thereof of the invention described above, the isolated nucleic acid of the present invention, the expression vector of the present invention, the cell of the present invention, the product (e.g., antibody and antigen-binding fragment thereof) produced by the method of the present invention, or the pharmaceutical composition of the present invention; the disease is preferably a tumor; and the tumor is preferably colorectal cancer.

In some embodiments, the present invention provides use of the antibody or antigen-binding fragment thereof described above, the isolated nucleic acid of the present invention described above, the expression vector of the present invention described above, the cell of the present invention described above, the product (e.g. antibody and antigen-binding fragment thereof) produced by the method of the present invention described above, or the pharmaceutical composition of the present invention described above in the manufacture of a medicament for the prevention and/or treatment of a disease associated with abnormal expression of PD-L1, the disease is preferably a tumor; and the tumor is preferably colorectal cancer.

In some embodiments, the present invention provides a kit, which comprises the antibody or antigen-binding fragment thereof of the present invention, the isolated nucleic acid of the present invention, the expression vector of the present invention, the cell of the present invention, or a product (e.g., antibody and antigen-binding fragment thereof) produced by the method of the present invention, and an instruction for use.

### Terms and Definitions:

Unless otherwise defined, terms used herein shall have the meanings generally understood by those of ordinary skill in the art. For terms explicitly defined herein, the meaning of the term shall be subject to the definition.

As use herein, the term "antibody" (Ab) refers to an immunoglobulin molecule that specifically binds to the target antigen or has immunoreactivity, including polyclonal, monoclonal, genetically engineered and other modified forms of antibodies (including but not limited to chimeric antibodies, humanized antibodies, fully human-origin antibodies, heterologous coupled antibodies (such as bispecific, trispecific and tetraspecific antibodies, diabodies, tribodies and tetrabodies), antibody conjugates) and antigen-binding fragments of antibodies (including, for example, Fab', F(ab')₂, Fab, Fv, rIgG and scFv fragments). In addition, unless otherwise defined, the term "monoclonal antibody" (mAb) is intended to include intact antibody molecules as well as incomplete antibody fragments (e.g. Fab and F (ab')₂ fragments, which lack the Fc fragment of the intact antibody (cleansed more quickly from the animal circulation) and therefore lack Fc-mediated effector function) capable of specifically binding to a target protein (see Wahl et al., J. Nucl. Med. 24: 316, 1983; the contents of which are incorporated herein by reference).

As use herein, the term "antigen-binding fragment" refers to one or more antibody fragments that retain the ability to specifically bind to a target antigen. The antigen binding function of an antibody can be performed by a fragment of the full-length antibody. The antibody fragment may be Fab, F(ab')₂, scFv, SMIP, diabody, tribody, affibody, nanobody, aptamer or single-domain antibody. Examples of binding fragments encompassing the term "antigen-binding fragment" of an antibody include, but are not limited to: (i) Fab fragment, a monovalent fragment consisting of VL, VH, CL and CH1 domains; (ii) F(ab)₂ fragment, a bivalent fragment comprising two Fab fragments linked by disulfide bonds in the hinge region; (iii) Fd fragment consisting of VH and CH1 domains; (iv) Fv fragment consisting of VL and VH domains of the single arm of the antibody; (V) dAb fragment comprising the VH and VL domains; (vi) dAb fragment consisting of a VH domain (Ward et al., Nature 341: 544-546, 1989); (vii) dAb fragment consisting of a VH or VL domain; (viii) isolated complementarity determining region (CDR); and (ix) a combination of two or more separated CDRs, which may optionally be connected by a synthetic linker. Furthermore, although the two domains VL and VH of the Fv fragment are encoded by separate genes, the two domains can be conjugated using a recombinant method by a linker that enables them to be made into a single protein chain in which the VL and VH regions are paired to form a monovalent molecule (referred to as single-chain Fv (scFv); see, for example, Bird et al., Science 242: 423-426, 1988 and Huston et al., Proc. Natl. Acad. Sci. USA 85: 5879-5883, 1988). These antibody fragments can be obtained by conventional techniques known to those of skill in the art, and these fragments are screened for use in the same manner as intact antibodies. Antigen-binding fragments may be produced by recombinant DNA techniques, enzymatic or chemical cleavage of intact immunoglobulin, or in some embodiments by chemical peptide synthesis procedures known in the art.

As use herein, the term "PD-L1" refers to programmed death ligand-1, also known as CD279 (differentiation cluster 279), which is an important immunosuppressive molecule. The PD-L1 is preferably human PD-L1.

As use herein, the term "anti-programmed death ligand-1 antibody", "programmed death ligand-1 antibody", "anti-PD-L1 antibody", "PD-L1 antibody", "anti-PD-L1 antibody moiety" and/or "anti-PD-L1 antibody fragment" and the like refer to any protein- or peptide-containing molecule comprising at least a portion of an immunoglobulin molecule capable of specifically binding to PD-L1 (for example, but not limited to at least one complementarity determining region (CDR) of a heavy or light chain or a ligand binding portion thereof, a heavy or light chain variable region, a heavy or light chain constant region, a framework region or any portion thereof). The PD-L1 antibody also includes antibody-like protein scaffolds (such as the tenth fibronectin type III domain (10Fn3)), which contain BC, DE and FG structural loops similar in structure and solvent accessibility to CDR of the antibody. The tertiary structure of the 10Fn3 domain is similar to the tertiary structure of the heavy chain variable region of IgG, and by replacing the residues of the BC, DE and FG loops of 10Fn3 with residues of the CDR-H1, CDR-H2 or CDR-H3 region from the PD-L1 monoclonal antibody, one skilled in the art can graft, for example, the CDR of the PD-L1 monoclonal antibody onto the fibronectin scaffold.

As use herein, the term "bispecific antibody" refers to an antibody, typically a human or humanized antibody, which has monoclonal binding specificity for at least two different antigens. In the present invention, one of the binding specificities may be detected for an epitope of PD-L1, and the other may be detected for another epitope of PD-L1 or any other antigen, such as a cell surface protein, a receptor, a receptor subunit, a tissue-specific antigen, a virus-derived protein, a virus-encoded envelope protein, a bacterial-derived protein or a bacterial surface protein, etc.

As use herein, the term "chimeric" antibody refers to an antibody which has a variable region of an immunoglobulin derived from one source organism, such as a rat or mouse, and a constant region of an immunoglobulin derived from a different organism, such as a human. Methods for producing chimeric antibodies are known in the art. See, for example, Morrison, 1985, Science 229 (4719): 1202-7; Oi et al., 1986, Bio Techniques 4: 214-221; Gillies et al., 1985 J Immunol Methods 125: 191-202; all of which are incorporated herein by reference.

As use herein, the term "complementarity determining region" (CDR) refers to a hypervariable region found in both light and heavy chain variable domains. The more conserved part of the variable domain is called the framework region (FR). As understood in the art, the amino acid position representing the hypervariable region of an antibody may vary depending on the context and various definitions known in the art. Some positions within the variable domain can be considered heterozygous hypervariable positions because these positions can be considered to be within the hypervariable region under a set of standards (such as IMGT or KABAT) and are considered to be outside the hypervariable region under a different set of standards (such as KABAT or IMGT). One or more of these positions may also be found in the extended hypervariable region. The present invention includes antibodies that contain modifications in these heterozygous hypervariable positions. The variable domains of the natural heavy and light chains each comprise four framework domains predominantly in a lamellar configuration, which are linked by three CDRs (CDR1, CDR2 and CDR3). These three CDRs form loops connecting the lamellar structure and in some cases form part of the lamellar structure. The CDRs in each chain are tightly held together by the FR regions in the order FR1-CDR1-FR2-CDR2-FR3-CDR3-FR4, and together with CDRs from other antibody chains contribute to the formation of antigen binding sites of antibodies (see Kabat et al., Sequences of Protein of Immunological Interest, National Institute of Health, Bethesda, Md. 1987; incorporated herein by reference). For example, as herein, CDR1-VH, CDR2-VH and CDR3-VH refer to the first CDR, the second CDR and the third CDR of the variable region of the heavy chain (VH), respectively, and these three CDRs constitute the CDR combination of the heavy chain (or its variable region) (VHCDR combination); CDR1-VL, CDR2-VL and CDR3-VL refer to the first CDR, the second CDR and the third CDR of the variable region of the light chain (VL), respectively, and these three CDRs constitute the CDR combination of the light chain (or its variable region) (VLCDR combination).

As use herein, the term "antibody conjugate" refers to a conjugate formed by chemically bonding an antibody molecule to another molecule directly or through a linker, for example, an antibody-drug conjugate (ADC), wherein the drug molecule is the other molecule.

As use herein, the term "monoclonal antibody" refers to an antibody derived from a single clone (including any eukaryotic, prokaryotic, or bacteriophage clone), and is not limited to the method by which the antibody is produced.

As use herein, the term "VH" refers to the variable region of the immunoglobulin heavy chain of an antibody (including the heavy chain of Fv, scFv or Fab). The term "VL" refers to the variable region of the immunoglobulin light chain (including the light chain of Fv, scFv, dsFv or Fab).

As use herein, the term "percentage (%) sequence identity" refers to the percentage of amino acid (or nucleotide) residues of a candidate sequence that are identical to those of a reference sequence after alignment of sequences and introduction of gaps (if desired) for maximum percentage sequence identity (e.g., for optimal alignment, gaps may be introduced in one or both of the candidate and reference sequences, and non-homologous sequences may be ignored for comparison purposes). For the purpose of determining the percentage sequence identity, the alignment can be achieved in a variety of ways well known to those of skill in the art, such as using publicly available computer software, such as BLAST, ALIGN, or Megalign (DNASTAIi) software. Those of skill in the art can determine appropriate parameters for measuring alignment, including any algorithms that require maximum alignment over the full length of the sequence being compared. For example, the reference sequence aligned for comparison with the candidate sequence may show that the candidate sequence exhibits from 50% to 100% sequence identity over the full length of the candidate sequence or over selected portions of successive amino acid (or nucleotide) residues of the candidate sequence. The length of the candidate sequences aligned for comparison may be, for example, at least 30% (e.g., 30%, 40%, 50%, 60%, 70%, 80%, 90%, or 100%) of the length of the reference sequence. When the position in the candidate sequence is occupied by the same amino acid (or nucleotide) residue as the corresponding position in the reference sequence, these molecules are the same at that position.

As use herein, that term "specific binding" refers to a binding reaction that determines the presence of an antigen in a heterogeneous population of proteins and other biomolecules which specifically recognized, for example, by antibodies or antigen-binding fragments thereof. Antibodies or antigen-binding fragments thereof that specifically bind to an antigen will bind to the antigen with a KD of less than 100nM. For example, an antibody or antigen-binding fragment thereof that specifically binds to an antigen will bind to the antigen with a KD of up to 100nM (e.g., between 1pM and 100nM). An antibody or antigen-binding fragment thereof that does not show specific binding to a particular antigen or epitope thereof will show a KD of greater than 100nM (e.g., greater than 500nM, 1µM, 100µM, 500µM or 1mM) for that particular antigen or epitope thereof. A variety of immunoassay methods can be used to select antibodies that perform specific immune response with specific proteins or carbohydrates. For example, solid-phase ELISA immunoassay is routinely used to select antibodies that perform specific immune response with proteins or carbohydrates. See Harlow & Lane, Antibodies, ALabortory Manual, Cold Spring Harbor Press, NewYork (1988) and Harlow & Lane, Using Antibodies, A Laboratory Manual, Cold Spring Harbor Press, NewYork (1999), which describe immunoassay methods and conditions that can be used to determine specific immunoreactivity.

As use herein, the term "vector" includes a nucleic acid vector, such as a DNA vector (e.g., a plasmid), an RNA vector, a virus, or other suitable replicon (e.g., a viral vector). Various vectors have been developed for delivering polynucleotides encoding foreign proteins into prokaryotic or eukaryotic cells. The expression vector of the present invention contains polynucleotide sequences and additional sequence elements, for example, for expressing proteins and/or integrating these polynucleotide sequences into the genome of mammalian cells. Certain vectors that can be used to express antibodies and antibody fragments of the present invention include plasmids containing regulatory sequences (such as promoter and enhancer regions) that direct gene transcription. Other useful vectors for expressing antibodies and antibody fragments contain polynucleotide sequences that enhance the translation rate of these genes or improve the stability or nuclear output of mRNA produced by gene transcription. These sequence elements include, for example, 5' and 3' untranslated regions, internal ribosomal entry sites (IRES) and polyadenylation signal sites to direct effective transcription of genes carried on expression vectors. The expression vector of the present invention may also contain polynucleotides encoding markers for selecting cells containing such vectors. Examples of suitable markers include genes encoding resistance to antibiotics, such as ampicillin, chloramphenicol, kanamycin or nourseothricin.

As use herein, the terms "subject" and "patient" refer to an organism receiving treatment for a particular disease or condition, such as cancer or infectious disease, as described herein. Examples of subjects and patients include mammals, such as humans, primates, pigs, goats, rabbits, hamsters, cats, dogs, guinea pigs, bovine family members (such as domestic cattle, bison, buffalo, elk, yak, etc.), cattle, sheep, horses, bison, etc., receiving treatment for diseases or conditions, such as cell proliferative disorders, for example, cancer or infectious diseases.

As use herein, that term "treatment" refers to surgical or therapeutic treatment, the purpose of which is to prevent, alleviate (reduce) the progression of undesirable physiological changes or lesions in the subject of treatment, such as the progression of cell proliferative disorders (e.g., cancer or infectious diseases). Beneficial or desired clinical outcomes include but not limited to, alleviation of symptoms, reduction of disease severity, stabilization (i.e., no deterioration) of the disease state, delay or amelioration of disease progression, improvement or mitigation of the disease state, and remission (whether partial or complete), whether detectable or undetectable. The subjects to be treated include those who already suffer from diseases or conditions, and those who are susceptible to diseases or conditions or intend to prevent diseases or diseases. When referring to terms such as alleviation, reduction, mitigation, amelioration and remission, the meanings also include elimination, disappearance and non-occurrence.

### BRIEF DESCRIPTION OF DRAWINGS

The foregoing and other aspects of the present invention will be clearly illustrated by the following detailed description of the invention and the accompanying figures. The accompany figures are intend to illustrate some preferred embodiments of the invention; however, it is to be understood that the invention is not limited to the particular embodiments disclosed.
FIG. 1. The titer of mouse serum binding to human PD-L1-mFc (A) and PD-L1-His (B) recombinant proteins determined after the last immunization.
FIG. 2. Fluorescence-activated Cell Sorting (FACS) and gating strategy diagram of PD-L1 specific B cells.
FIG. 3. Binding of PD-L1 protein to PD-1 protein blocked by anti-PD-L1 antibody as determine by competitive ELISA.
FIG. 4. EC50 of anti-PD-L1 antibody bound to PD-L1 protein on cell surface determined by FACS.
FIG. 5. Expression and activity of reporter genes increased by anti-PD-L1 antibody in Jurkat-PD-1-CHO-PD-L1-NFAT system.
FIG. 6. IFN-γ secretion promoted by anti-PD-L1 antibody in mixed lymphocyte reaction.
FIG. 7. Antibody-dependent cell-mediated cytotoxicity (ADCC) activity of anti-PD-L1 antibody against A431 cells.
FIG. 8. Growth of MC38-hPD-L1 colon cancer tumor inhibited by murine anti-PD-L1 antibody in human PD-1/PD-L1 transgenic mice.
FIG. 9. Growth of MC38-hPD-L1 colon cancer tumor inhibited by humanized anti-PD-L1 antibody in human PD-L1 transgenic mice.

### DETAILED DESCRIPTION

The present invention is described in detail below in conjunction with embodiments and accompanying figures, which are intended to illustrate some preferred embodiments of the invention. However, it is to be understood that the invention is not limited to the particular embodiments disclosed or considered as limitations to the scope of the invention. If no specific conditions are specified in the examples, the conventional conditions or the conditions suggested by the manufacturer shall be followed. If the manufacturer is not indicated, the reagents or instruments used are conventional products that can be purchased commercially.

### Example 1 Production of PD-L1 antibody by mouse immunization

Female SJL mice (purchased from Beijing Vital River Laboratory Animal Technology Co., Ltd.) or Balb/c mice (purchased from Shanghai SLAC Laboratory Animal Co., Ltd) aged 6-8 weeks were subjected to the first immunization with human PD-L1 protein fused with mouse Fc (PD-L1-mFc, Novoprotein, Cat. CM06, or Sino Biological, Cat. 10084-H05H) or human PD-L1-His (Novoprotein, Cat. C315 or Sino Biological, Cat: 10084-H08H) and complete Freund's adjuvant (CFA, Sigma, Cat. F5881). Mice were then subjected to the last three immunizations with the above PD-L1-mFc or human PD-L1-His and an incomplete Freund's adjuvant (IFA, Sigma, Cat. F5506) and unmethylated cytosine guanine dinucleotide (CpGODN1826, synthesized by Shanghai Sangon Biotech), and injected with 50µg of a uniform and stable emulsion prepared by emulsification operation per mouse each immunization. In particular, during the first and second immunizations, the antigen were injected into the hind foot pads and back, and during the third and fourth immunizations, the antigen were injected subcutaneously into back and near the tail, in order to obtain antiserum with high titer, high affinity and high specificity as well as specific immune cells. On the 5th-7th day after the last immunization (the fourth immunization), the mice were euthanized and the spleen was aseptically taken out. The spleen lymphocytes of mice were aseptically separated, extracted and aliquoted into cryopreservation tubes, which were then frozen in liquid nitrogen. Blood samples were collected 10 days after the second immunization, 10 days after the third immunization and the day of euthanasia, and the serum was separated. Enzyme-linked immunosorbent assay (ELISA) was used to determine the titer of anti-PD-L1 specific antibodies in the serum.

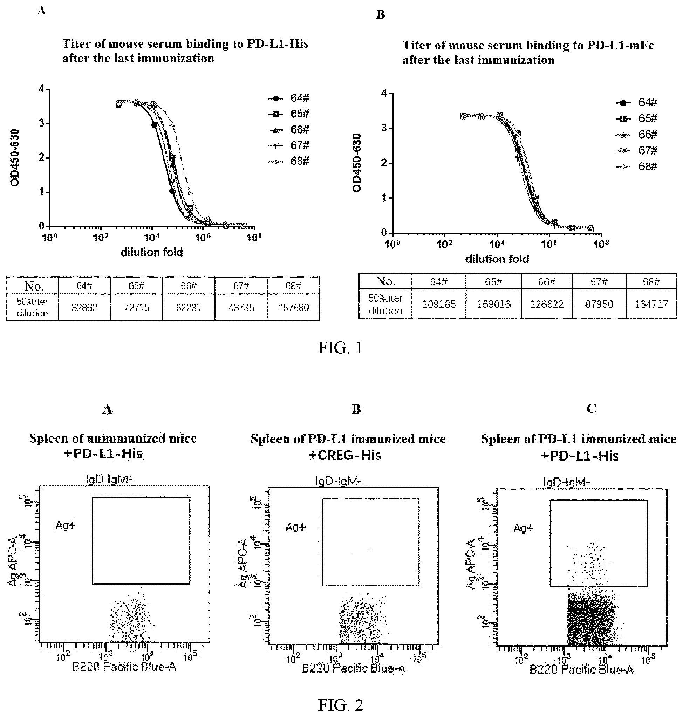

The results in FIG.1 show that after four immunizations, the titers of the antibodies binding to human PD-L1-mFc and PD-L1-His in the serum of immunized mice were very high. It shows that this method to immunize mice can be utilized to make mice produce high titer of anti-PD-L1 antibody.

### Example 2 Fluorescence-activated cell sorting (FACS) of PD-L1-specific single B cells

Spleen cells from mice immunized with PD-L1 protein were treated with the antigen PD-L1-His protein (Novoprotein, Cat. C315 or Sino Biological, Cat. 10084-H08H), the indirect marker antibody anti-His-APC (R&D Systems, Cat. IC050A) and the antibody against specific markers on the surface of mouse B cells (anti-mouse B220-Pacfic Blue, R&D Systems, Cat. 553089; Anti-mouse IgD-PE, R&D Systems, Cat. 558597; Anti-mouse IgM-PE Cy7, R&D Systems, Cat. 552867), and the dye 7-AAD (R&D Systems, Cat. 51-68981E) distinguishing dead from living cells was added before sorting. PD-L1-specific single B cells (7AAD⁻B220⁺IgD⁻IgM⁻PDL1-His⁺) were sorted into PCR wells containing cell lysate and RNase inhibitors by using AriaIII (BD Company) flow cytometry with one cell collected in each well. The results show (FIG. 2) that no significant PD-L1+ antigen-specific B cell subsets were detected in the spleen (A) of blank control mice without immunization or the spleen (B) of mice immunized with PD-L1 stained with the His-tagged unrelated protein CREG-His, while PD-L1+ B cells, about 114 PD-L1+ B cells per 10⁶ spleen cells were detected in the spleen (C) of mice immunized with PD-L1 labeled with PD-L1-His.

### Example 3 Amplification and high throughput expression of monoclonal antibody

The mRNA of single cells was reverse transcribed into cDNA using the method of Example 1 in Chinese Patent Application No. 201811618134.4, "Combined primers for nested amplification and application of combined primer". Then cDNA was used as template for nested PCR to amplify the heavy chain and light chain of the antibody, respectively. The heavy chain variable region and light chain variable region of the antibody were amplified and cloned into the expression vector for heavy chain and the expression vector for light chain by homologous recombination. The constant regions in the expression vector for heavy chain and the expression vector for light chain were both from human IgG1. The sequence for complete heavy chain expression was signal peptide-VH-CH1-hinge region-CH2-CH3, and the sequence for complete light chain expression was signal peptide-Vκ-Cκ. The cloning and expression of single B cell antibodies described above all achieve rapid identification and discovery of antibodies in 96-well plates in a high throughput manner. After a series of physicochemical and functional screening of the heavy chain and light chain of 324 cloned antibodies, a total of 9 candidate murine antibodies with physicochemical and functional activities equivalent to or better than those of the marketed PD-L1 antibody Avelumab or Atezolizumab were obtained, and the CDRs of their sequences were analyzed by IMGT and KABAT software, respectively. The corresponding sequence information is shown in Table 1 to 2 below, wherein Table 1 shows the VH and VL sequences of the murine antibodies, and Table 2 shows the results of IMGT and KABAT analysis of the murine antibodies.

**Table 1. Specific sequence information of heavy chain variable region and light chain variable region of murine anti-PD-L1 antibody**

| **Antibody No.** | **Sequence No.** | **Sequence of heavy chain variable region (VH)** |
|---|---|---|
| 34 | SEQ ID NO: 1 | EVQLQESGGDLVKPGGSLKLSCAASGFTFNTYGMSWVRQ TPDKRLEWVATLSSGGSYTYYSESVKGRFTISRDNAKNTL YLQMNSLKSEDTAIYYCARPTADWHLDVWGTGTTVTVS S |
| 50 | SEQ ID NO: 3 | EVQLQESGGDLVKPGGSLKLSCAASGFTFNTYGMSWVRQ TPDKRLEWVATLSSGGSYTYYSESVKGRFTISRDNAKNTL YLQMTSLKSEDTAIYYCARPTADWHLDVWGTGTTVTVSS |
| 90 | SEQ ID NO: 5 | EVQLQESGGDLVKPGGSLKLSCTASGFTFSTYGMSWVRQ TPDKRLEWVATVSSGGSYTYYPDSVKGRFTISRDNAKNT LYLQMSSLKSEDTAIYYCARPTADWHLDVWGTGTTVTVS S |
| 130 | SEQ ID NO: 7 | EVQLQESGPGLAKPSQTLSLTCSVTGYSITSDYWNWIRKF PGNKLEYVGYISYTGSTYYNPSLRSRISITRDTSKNQYYLQ LNSVTAEDTATYYCARCPGWLNAMDYWGQGTTVTVSS |
| 156 | SEQ ID NO: 9 | EVQLQESGPELVKPGASVKISCKASGYTFTDYYMNWVRQ SHGKSLEWIGDINPNNGDTSYNQKFKGKATLTVDKSSSTA YMDLRSLTSEDSAVYYCASSVMDYWGQGTTVTVSS |
| 370 | SEQ ID NO: 11 | EVQLQESGPGLAKPSQTLSLTCSVTGYSITSDYWNWIRKFP GNKLEYMGYISYTGSTYYNPSLKSRISIARDTSRNQYYLQ LNSVTTEDSATYYCARAGGWLLPFAVWGTGTTVTVSS |
| 373 | SEQ ID NO: 13 | EVQLQESGGGLVKPGGSLKLSCAASGFTFSDYGMHWVRQ APEKGLEWVAYISSGSSTIYYADTVKGRFTISRDNAKNTLF LQMTSLRSEDTAMYYCARRNFGSSYDYWGQGTTVTVSS |
| 413 | SEQ ID NO: 15 | EVQLQESGPGLAKPSQTLSLTCSVTGYSITSDYWNWIRKFP GNKLEYMGYISYTGSTYYNPSLKSRISIARDTSRNQYYLQ LKSVTTEDTATYYCARAGGWLLPFAVWGTGTTVTVSS |
| 794 | SEQ ID NO: 17 | EVQLQESGPSLVKPSQTLSLTCSVTGDSITSGYWNWIRKFP GNKLEYMGYISYSGSTYYNPFLKSRISITRDTSKNQYYLQL NSVTTEDTATYYCAKMGDWLAWFAYWGQGTTVTVSS |

| **Antibody No.** | **Sequence No.** | **Sequence of light chain variable region (VL)** |
|---|---|---|
| 34 | SEQ ID NO: 2 | DILMTQSPSSLSASLGGKVTITCNASQDINKYIAWYQHKPG KGPSLLIHYTSTLQPGIPSRFSGSGSGRDYSFSISNLEPEDIA TYYCLQHDNLLFTFGSGTKLEIK |
| 50 | SEQ ID NO: 4 | DIQMTQSPSSLSASLGGKVTITCKASQDINKYIAWYQHKP GKGPRLLIHYTSTLQPGIPSRFSGSGSGRDYSFSISNLEPEDI ATYYCLQHDNLLFTFGSGTKLEIK |
| 90 | SEQ ID NO: 6 | DIQMTQTPSSLSASLGGKVTITCKASQDINRYIAWYQHKP GKGPSLLIHYTSTLQPGIPSRFSGSGSGRDYSFSISNLEPEDI ATYYCLQHDNLLFTFGSGTKLEIK |
| 130 | SEQ ID NO: 8 | DILMTQSPSSLAVSVGEKVTLSCKSSQSLLYSNNQKNSLA WYQQKPGQSPKLLIYWASTGESGVPDRFTGSGSGTDFTLT ISSVKAEDLAVYYCQQYYGYPFTFGAGTKLEIK |
| 156 | SEQ ID NO: 10 | DIVLTQSPALMSASPGEKVTMTCSASSSVNYVYWYQQKP RSSPKPWIYLTFNLASGVPARFSGSGSGTSYSLTISSMEAE DAATYYCQQWSSNPLTFGAGTKLEIK |
| 370 | SEQ ID NO: 12 | DIVITQSPSSLAVSVGEKVTMSCKSSQSLLYSSNQQNSLAW YQQKPGQSPILLIYWASTRESGVPDRFTGGGSGTDFTLTISS VRAEDLAVYYCQQYYNYPWTFGGGTKLEIK |
| 373 | SEQ ID NO: 14 | EIVMTQSHKFMSTSVGDRVSITCKASQDVDTPVAWYQQK PGQSPRLLIYWASIRHTGVPDRFTGSGSGTDFTLTISNVQSE DLADYFCQQYSSYPLTFGSGTKLEIK |
| 413 | SEQ ID NO: 16 | DIVMTQSPSSLAVSVGEKVTMSCKSSQSLLYSSNQQNSLA WYQQKPGQSPILLIYWASTRESGVPDRFTGSGSGTDFTLTI SSVRAEDLAVYYCQQYYSYPWTFGGGTKLEIK |
| 794 | SEQ ID NO: 18 | EIVMTQSPSSLAVSVGEKVTLSCKSSQSLLYSSNQKNSLA WYQQKPGQSPKLLIYWASTRESGVPDRFTGSGSGTDFTLT ISSVKAEDLAVYYCQQYYGYPYTFGGGTKLEIK |

The CDRs of each antibody were analyzed by KABAT and IMGT software, respectively. The specific sequence information is shown in Table 2 below:

**Table 2. Specific sequence information of CDRs of murine antibodies by analysis of KABAT and IMGT software**

| **KABAT analysis** | | | | | | |
|---|---|---|---|---|---|---|
| **Antibody No.** | **Sequence No.** | **CDR1-HC** | **Sequence No.** | **CDR2-HC** | **Sequence No.** | **CDR3-HC** |
| 34 | SEQ ID NO: 19 | TYGMS | SEQ ID NO: 20 | TLSSGGSY TYYSESVK G | SEQ ID NO: 21 | PTADWHLD V |
| 50 | SEQ ID NO: 25 | TYGMS | SEQ ID NO: 26 | TLSSGGSY TYYSESVK G | SEQ ID NO: 27 | PTADWHLD V |
| 90 | SEQ ID NO: 31 | TYGMS | SEQ ID NO: 32 | TVSSGGSY TYYPDSVK G | SEQ ID NO: 33 | PTADWHLD V |
| 130 | SEQ ID NO: 37 | SDYWN | SEQ ID NO: 38 | YISYTGST YYNPSLRS | SEQ ID NO: 39 | CPGWLNA MDY |
| 156 | SEQ ID NO: 43 | DYYMN | SEQ ID NO: 44 | DINPNNGD TSYNQKFK G | SEQ ID NO: 45 | SSVMDY |
| 370 | SEQ ID NO: 49 | SDYWN | SEQ ID NO: 50 | YISYTGST YYNPSLKS | SEQ ID NO: 51 | AGGWLLPF AV |
| 373 | SEQ ID NO: 55 | DYGMH | SEQ ID NO: 56 | YISSGSSTI YYADTVK G | SEQ ID NO: 57 | RNFGSSYD Y |
| 413 | SEQ ID NO: 61 | SDYWN | SEQ ID NO: 62 | YISYTGST YYNPSLKS | SEQ ID NO: 63 | AGGWLLPF AV |
| 794 | SEQ ID NO: 67 | SGYWN | SEQ ID NO: 68 | YISYSGST YYNPFLKS | SEQ ID NO: 69 | MGDWLAW FAY |

| **Antibody No.** | **Sequence No.** | **CDR1-LC** | **Sequence No.** | **CDR2-LC** | **Sequence No.** | **CDR3-LC** |
|---|---|---|---|---|---|---|
| 34 | SEQ ID NO: 22 | NASQDIN KYIA | SEQ ID NO: 23 | YTSTLQP | SEQ ID NO: 24 | LQHDNLLF T |
| 50 | SEQ ID NO: 28 | KASQDIN KYIA | SEQ ID NO: 29 | YTSTLQP | SEQ ID NO: 30 | LQHDNLLF T |
| 90 | SEQ ID NO: 34 | KASQDIN RYIA | SEQ ID NO: 35 | YTSTLQP | SEQ ID NO: 36 | LQHDNLLF T |
| 130 | SEQ ID NO: 40 | KSSQSLLY SNNQKNS LA | SEQ ID NO: 41 | WASTGES | SEQ ID NO: 42 | QQYYGYPF T |
| 156 | SEQ ID NO: 46 | SASSSVN YVY | SEQ ID NO: 47 | LTFNLAS | SEQ ID NO: 48 | QQWSSNPL T |
| 370 | SEQ ID NO: 52 | KSSQSLLY SSNQQNS LA | SEQ ID NO: 53 | WASTRES | SEQ ID NO: 54 | QQYYNYP WT |
| 373 | SEQ ID NO: 58 | KASQDVD TPVA | SEQ ID NO: 59 | WASIRHT | SEQ ID NO: 60 | QQYSSYPL T |
| 413 | SEQ ID NO: 64 | KSSQSLLY SSNQQNS LA | SEQ ID NO: 65 | WASTRES | SEQ ID NO: 66 | QQYYSYP WT |
| 794 | SEQ ID NO: 70 | KSSQSLLY SSNQKNS LA | SEQ ID NO: 71 | WASTRES | SEQ ID NO: 72 | QQYYGYP YT |

| **IMGT analysis** | | | | | | |
|---|---|---|---|---|---|---|
| **Antibody No.** | **Sequence No.** | **CDR1-HC** | **Sequence No.** | **CDR2-HC** | **Sequence No.** | **CDR3-HC** |
| 34 | SEQ ID NO: 73 | GFTFNTY G | SEQ ID NO: 74 | LSSGGSYT | SEQ ID NO: 75 | ARPTADW HLDV |
| 50 | SEQ ID NO: 79 | GFTFNTY G | SEQ ID NO: 80 | LSSGGSYT | SEQ ID NO: 81 | ARPTADW HLDV |
| 90 | SEQ ID NO: 85 | GFTFSTY G | SEQ ID NO: 86 | VSSGGSYT | SEQ ID NO: 87 | ARPTADW HLDV |
| 130 | SEQ ID NO: 91 | GYSITSDY | SEQ ID NO: 92 | ISYTGST | SEQ ID NO: 93 | ARCPGWL NAMDY |
| 156 | SEQ ID NO: 97 | GYTFTDY Y | SEQ ID NO: 98 | INPNNGDT | SEQ ID NO: 99 | ASSVMDY |
| 370 | SEQ ID NO: 103 | GYSITSDY | SEQ ID NO: 104 | ISYTGST | SEQ ID NO: 105 | ARAGGWL LPFAV |
| 373 | SEQ ID | GFTFSDY | SEQ ID | ISSGSSTI | SEQ ID | ARRNFGSS |
| | NO: 109 | G | NO: 110 | | NO: 111 | YDY |
| 413 | SEQ ID NO: 115 | GYSITSDY | SEQ ID NO: 116 | ISYTGST | SEQ ID NO: 117 | ARAGGWL LPFAV |
| 794 | SEQ ID NO: 121 | GDSITSGY | SEQ ID NO: 122 | ISYSGST | SEQ ID NO: 123 | AKMGDWL AWFAY |

| **Antibody No.** | **Sequence No.** | **CDR1-LC** | **Sequence No.** | **CDR2-LC** | **Sequence No.** | **CDR3-LC** |
|---|---|---|---|---|---|---|
| 34 | SEQ ID NO: 76 | QDINKY | SEQ ID NO: 77 | YTS | SEQ ID NO: 78 | LQHDNLLF T |
| 50 | SEQ ID NO: 82 | QDINKY | SEQ ID NO: 83 | YTS | SEQ ID NO: 84 | LQHDNLLF T |
| 90 | SEQ ID NO: 88 | QDINRY | SEQ ID NO: 89 | YTS | SEQ ID NO: 90 | LQHDNLLF T |
| 130 | SEQ ID NO: 94 | QSLLYSN NQKNS | SEQ ID NO: 95 | WAS | SEQ ID NO: 96 | QQYYGYPF T |
| 156 | SEQ ID NO: 100 | SSVNY | SEQ ID NO: 101 | LTF | SEQ ID NO: 102 | QQWSSNPL T |
| 370 | SEQ ID NO: 106 | QSLLYSSN QQNS | SEQ ID NO: 107 | WAS | SEQ ID NO: 108 | QQYYNYP WT |
| 373 | SEQ ID NO: 112 | QDVDTP | SEQ ID NO: 113 | WAS | SEQ ID NO: 114 | QQYSSYPL T |
| 413 | SEQ ID NO: 118 | QSLLYSSN QQNS | SEQ ID NO: 119 | WAS | SEQ ID NO: 120 | QQYYSYP WT |
| 794 | SEQ ID NO: 124 | QSLLYSSN QKNS | SEQ ID NO: 125 | WAS | SEQ ID NO: 126 | QQYYGYP YT |

### Example 4 Humanization of antibody

Firstly, the classical "CDRs transplantation" method was used to humanize the antibody, specifically, the humanized antibody with the highest homology was selected based on sequence to provide the framework regions (FRs) of an antibody, and the antigen-binding fragment complementarity determination regions (CDRs) of the target antibody based on Kabat numbering were transplanted into FRs to form a humanized antibody. Secondly, in order to better maintain the activity and affinity of the antibody, based on the antibody structure modeling analysis (MOE software): 1). the amino acid residues such as the antibody framework regions located at the VH-VL interface, close to or directly interacting with CDRs were selected for reverse mutation, and such amino acid residues were important for maintaining the conformation of CDRs; 2) considering the immunogenicity, the amino acid embedded in the protein was selected as far as possible for reverse mutation; 3) considering antibody stability and expression level, the mutation with molecular energy reduction was given priority; and 4). in the process of humanization, the affinity of humanized antibody was further improved by site-directed mutagenesis of amino acids in CDRs. By testing the affinity of humanized antibodies containing different mutations with human PD-L1 and the binding to cells expressing PD-L1 on the surface, humanized antibodies with the affinity, antibody characterization and activity function equivalent to or better than those of murine PD-L1 antibodies were screened.

Among them, CDRs of the preferred candidate antibodies after humanization of the PDL1-156 antibody were analyzed by IMGT and KABAT software as follows, and the corresponding sequence information is shown in Table 3 and Table 4 below, wherein Table 3 shows the VH and VL sequences of the humanized antibodies, and Table 4 shows the IMGT and KABAT analysis results of the humanized antibodies.

**Table 3. Specific sequence information of heavy chain variable region and light chain variable region of humanized anti-PD-L1 antibodies**

| **Antibody No.** | **Sequence No.** | **Sequence of heavy chain variable region (VH)** |
|---|---|---|
| 156-1H | SEQ ID NO: 127 | QVQLVQSGPELKKPGASVKISCKASGYTFTDYYMNWVR QAPGQSLEWIGDIWPNNGDTWYNQKFKGRVTLTRDTSTS TVYMELRSLRSEDTAVYYCARSVMDYWGQGTLVTVSS |
| 156-7H | SEQ ID NO: 129 | QVQLVQSGPELKKPGASVKISCKASGYTFTDYYMNWVR QAPGQSLEWIGDIWPNNGDTSYNQKFKGRVTLTRDTSTS TVYMELRSLRSEDTAVYYCARSVMDTWGQGTLVTVSS |
| 156-10H | SEQ ID NO: 131 | QVQLVQSGPELKKPGASVKISCKASGYTFTDYYMNWVR QAPGQSLEWIGDIWPNNGDTSYNQKFKGRVTLTRDTSTS TVYMELRSLRSEDTAVYYCARSVMSDWGQGTLVTVSS |
| 156-11H | SEQ ID NO: 133 | QVQLVQSGPELKKPGASVKISCKASGYTFTDYYMNWVR QAPGQSLEWIGDIWPNNGDTSYNQKFKGRVTLTRDTSTS TVYMELRSLRSEDTAVYYCARSVMDYWGQGTLVTVSS |

| **Antibody No.** | **Sequence No.** | **Sequence of light chain variable region (VL)** |
|---|---|---|
| 156-1H | SEQ ID NO: 128 | EIVLTQSPALLSLSPGERVTLSCSASSSVNYVYWYQQKPG QAPRPLIYLTFNLASGIPARFSGSGSGTDFTLTISSLEPEDFA VYYCQQWSVNPLTFGGGTKVEIK |
| 156-7H | SEQ ID NO: 130 | EIVLTQSPALLSLSPGERVTLSCSASSSVNYVYWYQQKPG QAPRPLIYLTFNLASGIPARFSGSGSGTDFTLTISSLEPEDFA VYYCQQWSVNPLTFGGGTKVEIK |
| 156-10H | SEQ ID NO: 132 | EIVLTQSPALLSLSPGERVTLSCSASSSVNYVYWYQQKPG QAPRPLIYLTFNLASGIPARFSGSGSGTDFTLTISSLEPEDFA VYYCQQWSSNPLTFGGGTKVEIK |
| 156-11H | SEQ ID NO: 134 | EIVLTQSPALLSLSPGERVTLSCSASSSVNYVYWYQQKPG |
| | | QAPRPLIYLTFNLASGIPARFSGSGSGTDFTLTISSLEPEDFA VYYCQQWSVNPLTFGGGTKVEIK |

The CDRs of each humanized antibody were analyzed by KABAT and IMGT software, respectively. The specific sequence information is as follows:

**Table 4. Specific sequence information of CDRs of each humanized PD-L1 antibody by analysis of KABAT and IMGT software**

| **KABAT analysis** | | | | | | |
|---|---|---|---|---|---|---|
| **Antibody No.** | **Sequence No.** | **CDR1-HC** | **Sequence No.** | **CDR2-HC** | **Sequence No.** | **CDR3-HC** |
| 156-1H | SEQ ID NO: 135 | DYYMN | SEQ ID NO: 136 | DIWPNNGD TWYNQKFK G | SEQ ID NO: 137 | SVMDY |
| 156-7H | SEQ ID NO: 141 | DYYMN | SEQ ID NO: 142 | DIWPNNGD TSYNQKFK G | SEQ ID NO: 143 | SVMDT |
| 156-10H | SEQ ID NO: 147 | DYYMN | SEQ ID NO: 148 | DIWPNNGD TSYNQKFK G | SEQ ID NO: 149 | SVMSD |
| 156-11H | SEQ ID NO: 153 | DYYMN | SEQ ID NO: 154 | DIWPNNGD TSYNQKFK G | SEQ ID NO: 155 | SVMDY |

| **Antibody No.** | **Sequence No.** | **CDR1-LC** | **Sequence No.** | **CDR2-LC** | **Sequence No.** | **CDR3-LC** |
|---|---|---|---|---|---|---|
| 156-1H | SEQ ID NO: 138 | SASSSVN YVY | SEQ ID NO: 139 | LTFNLAS | SEQ ID NO: 140 | QQWSVNPL T |
| 156-7H | SEQ ID NO: 144 | SASSSVN YVY | SEQ ID NO: 145 | LTFNLAS | SEQ ID NO: 146 | QQWSVNPL T |
| 156-10H | SEQ ID NO: 150 | SASSSVN YVY | SEQ ID NO: 151 | LTFNLAS | SEQ ID NO: 152 | QQWSSNPL T |
| 156-11H | SEQ ID NO: 156 | SASSSVN YVY | SEQ ID NO: 157 | LTFNLAS | SEQ ID NO: 158 | QQWSVNPL T |

| **IMGT analysis** | | | | | | |
|---|---|---|---|---|---|---|
| **Antibody No.** | **Sequence No.** | **CDR1-HC** | **Sequence No.** | **CDR2-HC** | **Sequence No.** | **CDR3-HC** |
| 156-1H | SEQ ID NO: 159 | GYTFTDY Y | SEQ ID NO: 160 | IWPNNGDT | SEQ ID NO: 161 | ARSVMDY |
| 156-7H | SEQ ID | GYTFTDY | SEQ ID | IWPNNGDT | SEQ ID | ARSVMDT |
| | NO: 165 | Y | NO: 166 | | NO: 167 | |
| 156-10H | SEQ ID NO: 171 | GYTFTDY Y | SEQ ID NO: 172 | IWPNNGDT | SEQ ID NO: 173 | ARSVMSD |
| 156-11H | SEQ ID NO: 177 | GYTFTDY Y | SEQ ID NO: 178 | IWPNNGDT | SEQ ID NO: 179 | ARSVMDY |

| **Antibody No.** | **Sequence No.** | **CDR1-LC** | **Sequence No.** | **CDR2-LC** | **Sequence No.** | **CDR3-LC** |
|---|---|---|---|---|---|---|
| 156-1H | SEQ ID NO: 162 | SSVNY | SEQ ID NO: 163 | LTF | SEQ ID NO: 164 | QQWSVNPL T |
| 156-7H | SEQ ID NO: 168 | SSVNY | SEQ ID NO: 169 | LTF | SEQ ID NO: 170 | QQWSVNPL T |
| 156-10H | SEQ ID NO: 174 | SSVNY | SEQ ID NO: 175 | LTF | SEQ ID NO: 176 | QQWSSNPL T |
| 156-11H | SEQ ID NO: 180 | SSVNY | SEQ ID NO: 181 | LTF | SEQ ID NO: 182 | QQWSVNPL T |

### Example 5 Determination of antibody purity by molecular exclusion chromatography

The purity of antibody was determined by molecular exclusion chromatography using TSKgel G3000SWXL column (TOSOH, 0008541) and pre-column TSKgel guard column SWXL (TOSOH, Cat. 0008543). The mobile phase was phosphate buffer solution (NaH₂PO₄-Na₂HPO₄), which was prepared by mixing 8.88g of NaH₂PO₄•2H₂O and 33.33g of Na₂HPO₄•12H₂O. The mobile phase was used to balance the chromatographic column, and the flow rate was 1mL/min. After the baseline was flattened, the sample was injected at a volume of 10µL. The ultraviolet detection wavelength was 280nm, the bandwidth was 16nm, and the reference wavelength was off. The determination results are shown in Table 5.

**Table 5. Purity of humanized anti-PD-L1 antibody**

| **Sample Number** | **Main peak (%)** | **High molecular weight peak (%)** | **Degradation (%)** |
|---|---|---|---|
| PDL1-156 | 95.03 | 1.33 | 2.88 |
| 156-1H | 96.16 | 3.00 | 0.84 |
| 156-7H | 81.80 | 3.56 | 14.64 |
| 156-10H | 86.05 | 3.5 | 10.45 |
| 156-11H | 93.17 | 6.83 | / |

### Example 6 KD determination of antibody binding to human and cynomolgus monkey PD-L1 recombinant protein

Biacore T200 (GE Healthcare) was used to determine binding affinity of the PD-L1 antibody to the human and cynomolgus monkey PD-L1-His proteins. Anti-human IgG Fc (Genway, Cat. GWB-20A705) was immobilized on CM5 chip (GE Healthcare, Cat. BR-1005-30) at 25°C. Anti-human Fc (Genway, Cat. GWB-20A705) was diluted to 20µg/ml with Acetate pH5.0 (GE Healthcare, BR-1003-51). The Amine method in the Immobilization method was used to immobilize. Alternatively, a commercially available Protein A (GE Healthcare, Cat. 29127556) chip was used for detection. The affinity between antibody and antigen was determined by multi-cycle kinetic method at 25°C. In each cycle, the antibody to be tested was captured on the fixed CM5 chip, then injected with recombinant human PD-L1-His (Novoprotein, Cat. 315) and cynomolgus monkey PD-L1-His protein (Sino Biological, Cat. 90251-C08H), and finally regenerated with Glycine pH1.5. The mobile phase was HBS-EP + Buffer (GE Healthcare, Cat. BR-1006-69), the flow rate was 30µL/min, and the binding time was 300 seconds. The regeneration flow rate was 30µL/min and the time was 30 seconds. Using Biacore T200 evaluation software (version 3.0), a 1:1 binding model was used to analyze the experimental data, fit the equilibrium dissociation constant KD of the antibody and antigen, and determine the binding rate constant Ka and dissociation rate constant Kd.

From the results, it can be seen that the binding of the tested PD-L1 antibodies to the human PD-L1 recombinant protein all showed an affinity of nM or better, and the affinity for the cynomolgus monkey PD-L1 recombinant protein was between 62.5 nM and 0.375 nM. See Table 6 below for details.

**Table 6. Determination result of Biacore binding affinity KD of murine PD-L1 antibody (M)**

| **Antibody No.** | **Human PD-L1** | **Cynomolgus monkey PD-L1** |
|---|---|---|
| PDL1-156 | 2.91 E-10 | 3.75 E-10 |
| PDL1-794 | 1.25 E-09 | 8.83 E-10 |
| PDL1-130 | 2.18 E-09 | 1.95 E-09 |
| PDL1-34 | 4.58 E-10 | 5.43 E-09 |
| PDL1-50 | 6.99 E-10 | 3.31 E-08 |
| PDL1-90 | 8.70 E-10 | 6.25 E-08 |
| PDL1-370 | 2.42 E-09 | 2.18 E-09 |
| PDL1-373 | 2.45 E-09 | 3.64 E-09 |
| PDL1-413 | 2.09 E-09 | 2.76 E-09 |

Table 7 shows that the binding of humanized antibodies 156-1H, 156-7H, 156-10H and 156-11H derived from murine antibody PDL1-156 to human PDL1 protein exhibited an affinity equivalent to PDL1-156.

**Table 7. Determination result of Biacore binding affinity of humanized PD-L1 antibody**

| **Antibody No.** | **Binding rate constant ka (1/Ms)** | **Dissociation rate constant kd (1/s)** | **Equilibrium dissociation constant KD (M)** |
|---|---|---|---|
| PDL1-156 | 8.02 E+05 | 2.33 E-04 | 2.91 E-10 |
| 156-1H | 4.940 E+05 | 2.265 E-04 | 4.584 E-10 |
| 156-7H | 5.184 E+05 | 1.805 E-04 | 3.481 E-10 |
| 156-10H | 5.954 E+05 | 1.333 E-04 | 2.239 E-10 |
| 156-11H | 5.742 E+05 | 1.928 E-04 | 3.359 E-10 |

### Example 7 ICso determination of antibody blocking the interaction between PD-L1 and PD-1

The IC₅₀ of anti-PD-L1 antibody blocking the binding of PD-L1 protein to PD-1 protein was determined by competitive ELISA.

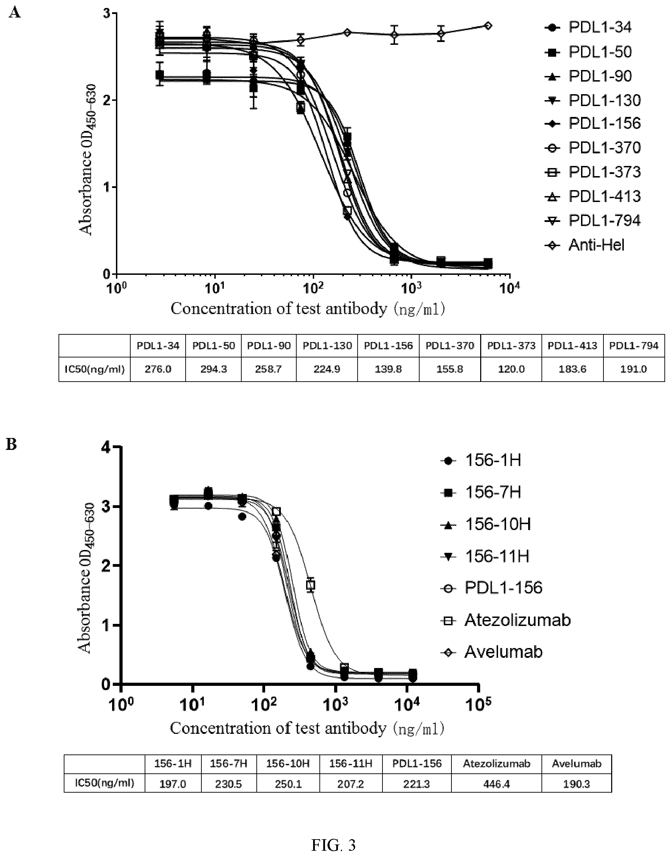

The recombinant human PD-L1 protein (Sino Biological, Cat. 10084-H05H) was diluted with carbonate buffer and added to a 96-well plate at a final concentration of 1µg/ml. After blocking with PBS solution containing 3% BSA, serially diluted anti-PD-L1 antibody (6000ng/ml-2ng/ml) and human PD-1-His recombinant protein (Sino Biological, Cat. 10377-H08H) were added for co-incubation. Then HRP-labeled anti-His tag antibody (MBL, Cat. D291-7) was added, and color development was performed with TMB. After termination with 1M sulfuric acid, OD values (dual wavelength 450nm-630nm) were read. The competitive binding curve of the test antibody can be drawn by matching the antibody concentration with the OD value to calculate the IC50 value. FIG. 3 shows the competitive binding curve of anti-PD-L1 antibody to human PD-L1 recombinant protein. The results show that the 9 murine anti-PD-L1 antibodies (A) and 4 humanized antibodies (B) tested could effectively block the interaction between human PD-L1 protein and human PD-1 protein compared with the antibody isotype negative control anti-Hel (prepared by Biointron) without any blocking effect, and the humanized antibodies had a comparable inhibitory activity to the murine PDL1-156 before humanization (B), with IC₅₀ of 197.0 ng/mL (156-1H), 230.5 ng/mL (156-7H), 250.1 ng/mL (156-10H) and 207.2 ng/mL (156-11H), respectively. The IC₅₀ of murine PD-L1-156 was 221.3 ng/ml, the positive control Atezolizumab (prepared by Biointron) was 446.4 ng/ml, and Avelumab (Pfizer, lot AU020322) was 190.3 ng/ml.

### Example 8 EC₅₀ determination of PD-L1 antibody binding to PD-L1 on cell surface by FACS

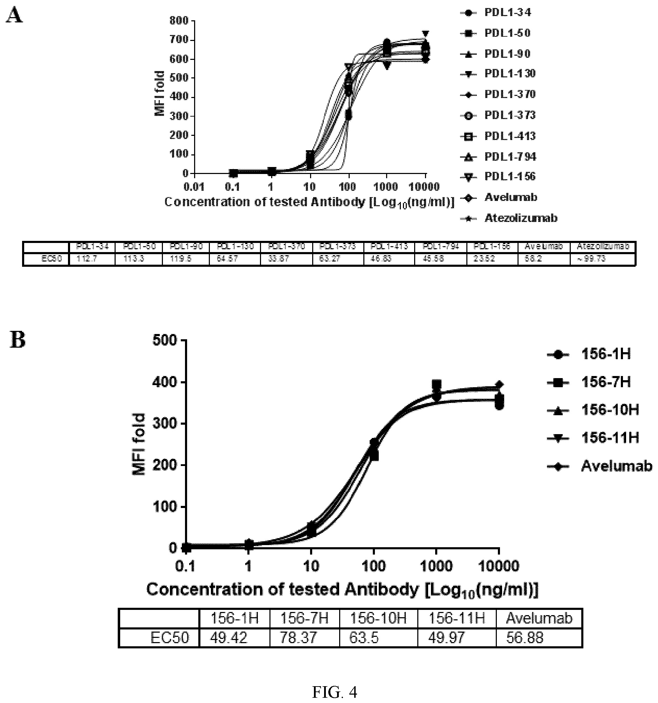

A gradient concentration of antibodies to be detected (final concentration of antibody: 10000ng/ml-0.1ng/ml, 10-foldserial dilution) were incubated with CHO-PD-L1 cells (Nanjing Yongshan Biotechnology Co., Ltd., 10⁵ cells/well) with high expression of PD-L1 on the cell surface at 4°C °C for 30min. After incubation, 1:250 diluted anti-human IgG PE fluorescent antibody (eBioscience, Cat. 12-4998-8) was added, and incubated at 4°C for 30min. The fluorescent antibody specifically bound to the Fc fragment of the antibody to be detected. The fluorescent intensity of PE was detected by FACS, and the ability of the antibody to be detected to bind to PD-L1 protein highly expressed on the cell surface was analyzed. As shown in FIG. 4, the EC₅₀ of the murine PD-L1 antibody to be test was similar to that of the positive controls Avelumab (EC₅₀ of 58.2 ng/ml) and Atezolizumab (~ 99.73 ng/ml) in this experiment, among which the EC₅₀ of PDL1-156 and PDL1-370 were the lowest, and the EC₅₀ of the detected humanized antibodies 156-1H, 156-7H, 156-10H and 156-11H were 49.42 ng/ml, 78.37 ng/ml, 63.5 ng/ml and 49.97 ng/ml, respectively, similar to that of the positive control Avelumab (EC₅₀ of 56.88 ng/ml) in this experiment. This determination quantitatively confirmed the ability of each PD-L1 antibody to bind to PD-L1 targets on the cell surface in a dose-dependent manner. MFI fold = MFI value of the experimental group/MFI value of the control group without drugs.

### Example 9 Determination of anti-PD-L1 antibody inhibiting PD-1: PD-L1 binding and signal transduction by PD-1/PD-L1-NFAT reporter gene assay

The antagonistic effects of PD-L1 antibody on PD-1/PD-L1 protein interaction and its signaling pathway were compared by using Jurkat cell line (GenScript, Cat.00612) stably transfected with PD-1 and CHO cell line (GenScript, Cat. M00613) stably transfected with PD-L1. When the inhibitory signaling pathway was inhibited, the expression of NFAT-controlled luminescence reporter gene increased, and the luminescence signal value increased. The blocking effect of antibody on PD-L1 was reflected by the intensity of relative luminescence units (RLUs).

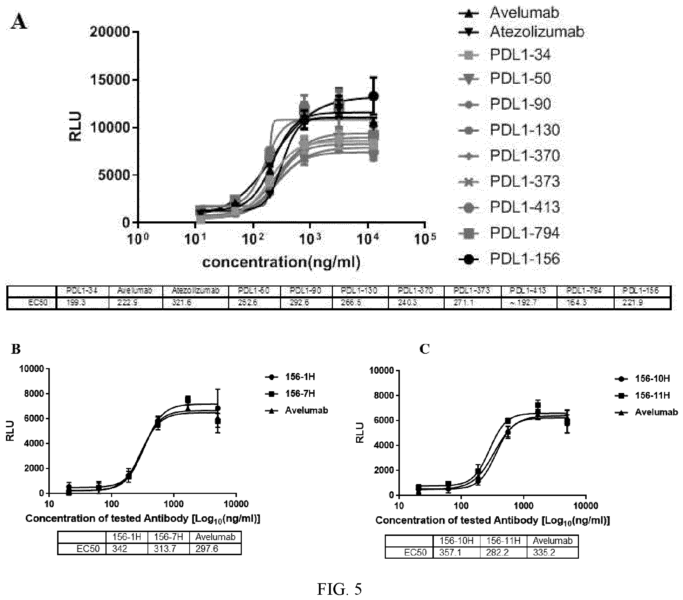

The CHO cells stably expressing PD-L1 were seeded on a 96-well white bottom plate with 40,000 cells/well and 100µL/well, and placed back into an incubator overnight. On the next day, the well plate was taken out, the culture medium was removed, and then the cells stably expressing PD-1 and the PD-L1 antibody to be tested were added for co-incubation. The PD-1 cells were added at 16,000 cells/well, while the antibody was serially diluted, added with each dose in triplicate at a volume of 100µL/well, and incubated for 6 hours. After the incubation, the well plate was taken out and added with luminescence detection reagent in the same volume (100µL). Then the values were read. According to the values, a regression curve was plotted by Graphpad with the 4-parameter analysis, and the EC₅₀ values of each antibody were obtained. The results are detailed in FIG.5A. The EC₅₀ values of the tested murine PD-L1 antibody were all similar to those of the positive control antibodies Avelumab and Atezolizumab (222.9 ng/ml and 321.6 ng/ml). FIGs.5B and 5C show that the EC₅₀ of the four humanized PD-L1 antibodies 156-1H, 156-7H and 156-10H, 156-11H were: 342ng/ml, 313.7 ng/ml, 357.1 ng/ml and 282.2 ng/ml, respectively, which were similar to the EC₅₀ of the positive control Avelumab. The determination quantitatively confirmed that murine and humanized anti-PD-L1 antibodies showed a dose-dependent suppression of T cell activity inhibition caused by the interaction of PD-1: PD-L1 on the cell surface, thus dose-dependently enhancing the activity of reporter gene in Jurkat cells.

### Example 10 ELISA detection of IFN-γ secreted by T cell in mixed lymphocyte reaction

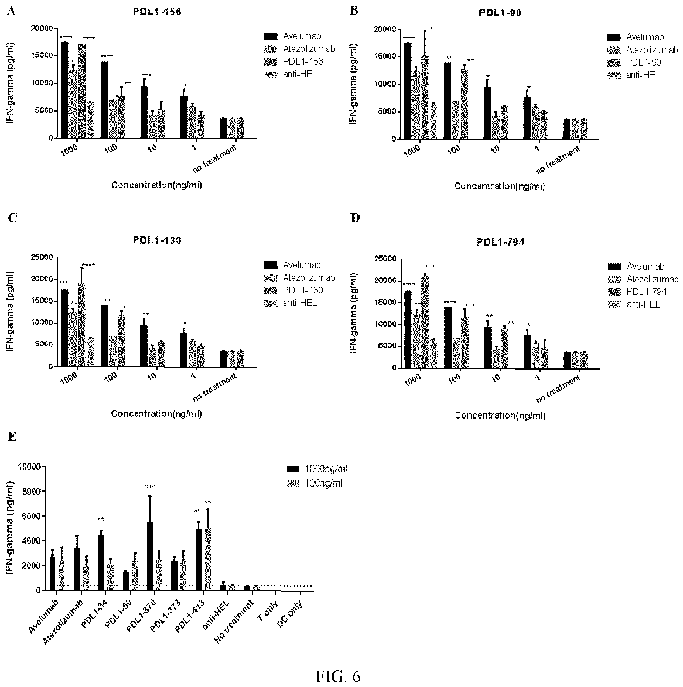

The activity of T cells enhanced by PD-L1 monoclonal antibody was determined by mixed lymphocyte reaction (MLR). CD14⁺ monocyte was isolated from peripheral blood mononuclear cells (PBMCs) of healthy human donor 1, and then induced *in vitro* to differentiate into dendritic cells (DCs) with recombinant human granulocyte-macrophage colony stimulating factor (GM-CSF, Peprotech, Cat. 300-03) and recombinant human interleukin-4 (rhIL-4, Peprotech, Cat. 200-04). On the 6th day of culture, LPS (Sigma, Cat: L4516) was added to stimulate DCs maturation. On the 7th day, DCs from donor 1 were mixed and co-cultured with CD4⁺ T cells enriched from PBMCs of healthy donor 2 at a ratio of DCs: CD4⁺ T cells being 1:10. The antibody to be tested or positive control antibody Avelumab or Atezolizumab (antibody concentration of 1000ng/ml, 100ng/ml, 10 ng/ml, 1ng/ml) was added to incubate for 4 days. After 4 days, the cell culture supernatant was collected, and the concentration of IFN-γ in the supernatant was determined by ELISA. As shown in FIG.6, all the tested murine antibodies and positive control antibodies Avelumab and Atezolizumab can significantly enhance the ability of CD4⁺ T cells to secrete IFN-γ in the MLR experiment compared with anti-HEL monoclonal antibody and no treatment negative control group, and as the concentration of PD-L1 antibody decreased, the activity of increasing IFN-secretion also decreased. The results indicated that PD-L1 antibody could enhance the function of T cells in a dose-dependent manner. T-test, ^{∗} P < 0.05, ^{∗∗} P < 0.01, ^{∗∗∗} P < 0.001, ^{∗∗∗∗} P < 0.0001.

### Example 11 Determination of Antibody Dependent Cell-mediated Cytotoxicity (ADCC) Activity of Anti-PD-Ll Antibody

The ADCC activity mediated by the anti-PD-L1 monoclonal antibody was determined by the cell killing experiment of natural killer (NK) cells from isolated and purified PBMCs of normal human on A431 cells with high expression of human PD-L1.

Preparation of effector cells: Cryopreserved human PBMC cells (StemExpress) were thawed and incubated overnight in RPMI1640 medium (Invitrogen, Cat. 11835030) supplemented with 100IU/ml recombinant human interleukin 2 (rhIL-2, Peprotech, Cat. 200-02). After that, the cells were collected and the living cells were counted. NK cells were then purified from PBMCs using the NK cell magnetic bead separation kit (Stemcell, Cat. 17955), resuspended in DMEM (Invitrogen, Cat. 11965084) supplemented with 10% inactivated fetal bovine serum and counted for use as effector cells.

Preparation of target cells: The target cells A431 were cultured in DMEM medium containing 10% fetal bovine serum. The day before the ADCC experiment, IFN-γ (Peprotech, Cat. 300-02) with the final concentration of 500IU was added to stimulate cells overnight.

The anti-PD-L1 antibody was diluted with DMEM medium containing 10% fetal bovine serum. The diluted antibody was added into white wall 96-well plates (Corning, Cat. 3610) at 25 µl/well, and target cells were added to the wells (25 µL/well, 10000 cells/well). The plates were incubated at an incubator at 37°C and 5% CO₂ for 30 minutes. After that, the effector cells were added to the wells (25 µL/well, 40,000 cells/well, i.e., the effector-target ratio NK: A431 = 4:1), with a total volume of 100 µL, and the plates were incubated at an incubator at 37°C and 5% CO₂ for 4 hours.

It is necessary to set the medium background control well (adding 100 µL of medium to the well), the target cell spontaneous death release well (only target cells in the well), the effector cell spontaneous death release well (only NK cells in the well), and the target cell maximum death release well (target cells plus the lysis buffer provided in the CytoTox-Glo^{™} Cytotoxicity kit (Promega, Cat. G9291)). Finally, the volume of all control wells was supplemented to 100 µL with medium. After incubation for 4 hours, the plates were taken out, 50µL of CytoTox-Glo^{™} CytoToxicity Assay Reagent was added to each well, mixed evenly on a shaking table, and left at room temperature for 15 minutes before reading chemiluminescence value (RLU).

The cell lysis rate caused by ADCC activity was calculated according to the following procedure: first, the average reading value of the medium background control well was subtracted from the reading value of all wells, and then the lysis rate% was calculated by: lysis rate%= (reading value of experimental well - reading value of target cell spontaneous death release well - reading value of effector cell spontaneous death release well) / (reading value of target cell maximum death release well - reading value of target cell spontaneous death release well) ^{∗} 100%

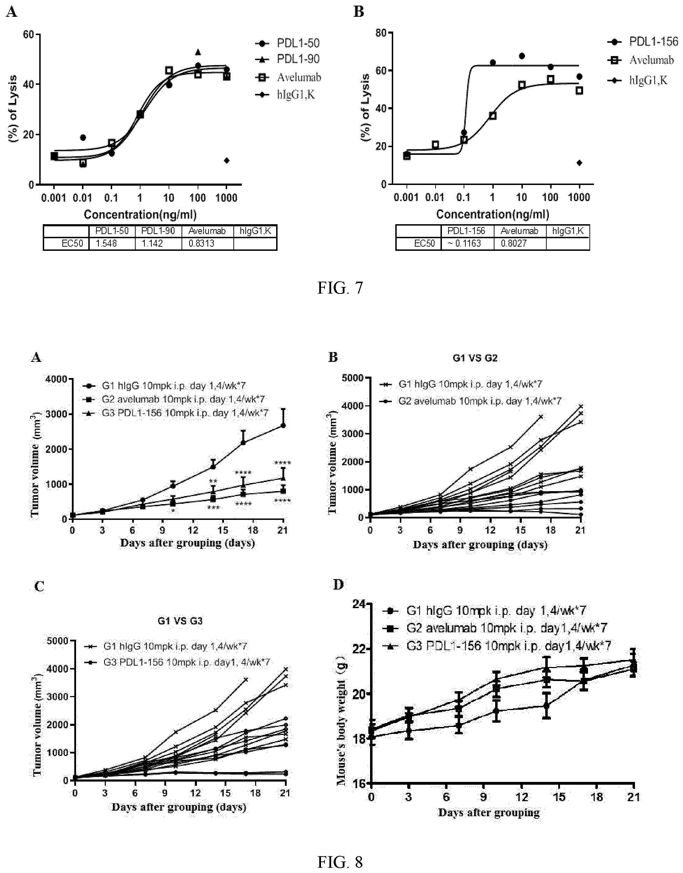

The results are shown in FIG.7. The experimental results show that the three murine PD-L1 antibodies tested and the FDA-approved positive control anti-PD-L1 drug Avelumab all showed antibody concentration-dependent lytic killing activity and similar EC50 values against the target cells A431. Also, the negative isotype control antibody hIgG1, K (Abdserotec, PHP010) showed no ADCC activity even at the highest concentration.

### Example 12 In vivo pharmacodynamics detection of murine PD-L1 antibody PDL1-156

B-hPD-1/hPD-L1 transgenic mice (Beijing Biocytogen Gene Biotechnology Co., Ltd.) were used to establish an animal model of MC38-hPD-L1 colon cancer, and the pharmacodynamics of PD-L1 antibody was then evaluated. 5 × 10⁵ MC38-hPD-L1 colon cancer cells were inoculated subcutaneously on the right side of mice. When the tumor volume reached about 110 mm³, mice with moderate individual tumor volume were selected into the group, and the animals were assigned to three experimental groups according to the tumor volume by random grouping software: anti-Hel hIgG isotype control group, anti-PDL1-156 antibody group, and positive drug Avelumab group (Pfizer, lot AU020322), with 8 mice in each group. Administration was started on the day of grouping (defined as the 0th day of the study). Each group was given 10 mg/kg by intraperitoneal injection twice a week for a total of 7 times. The tumor volume and body weight of mice were measured twice a week, and the measured values were recorded. The tumor volume (long diameter × short diameter²/2) and the tumor growth inhibition rate (TGI_{TV} (%) = [1-(Ti-T0)/(Vi-V0)] × 100%; Ti: the mean value of tumor volume in the treatment group on the day i of administration, T0: the mean value of tumor volume in the treatment group on the 0th day of administration; Vi: the mean value of tumor volume in the solvent control group on the day i of administration, V0: the mean value of tumor volume in the solvent control group on the 0th day of administration) were calculated. Also, the tumor volume was statistically analyzed, and P < 0.05 was considered as significant difference.

On the 21st day of grouping and administration, compared with the control group, the positive drug Avelumab group and PDL1-156 murine anti-PD-L1 antibody group had significant and similar inhibitory effects on tumor volume, and there was a statistical difference (P < 0.05). See FIGs. 8A, B, C, and Table 8.

**Table 8. Effect of test substance on volume of MC38-hPD-L1 tumor from B-hPD-1/hPD-L1 humanized mouse**

| **Group** | **Test substance** | **Tumor volume (mm³) ^{a}** | | | **P^{b}** |
|---|---|---|---|---|---|
| | | **Before administration** | **Day 21 of grouping and administration** | **TGI_{TV} (%)** | |
| G1 | hIgG | 114 ± 3 | 2675 ± 470 | - | - |
| G2 | Avelumab | 114 ± 3 | 797 ± 176 | 73.3 | ^{∗∗}0.001 |
| G3 | PDL1-156 | 114 ± 3 | 1173 ± 291 | 58.6 | ^{∗} 0.014 |

| | | | | | |
|---|---|---|---|---|---|
| Note: a: mean ± standard error; B: the tumor volume of the administration group and the tumor volume of the hIgG control on the 21st day after the grouping and administration were statistically analyzed, two-way ANOVA test, ^{∗} p < 0.05, ^{∗∗} p < 0.01, ^{∗∗∗} p < 0.001, ^{∗∗∗∗} p < 0.0001. | | | | | |

In addition, during the experiment, one mouse in hIgG control group died abnormally in advance. Except for that, the rest of the experimental animals were in good activity and eating state during the administration, and their weight increased to a certain extent. The results showed that the antibody was safe, as shown in FIG. 8D and Table 9.

**Table 9. Effect of test substance on body weight of β-hPD-1/hPD-L1 humanized mice transplanted with MC38-hPD-L1 tumor cells**

| **Group** | **Test substance** | **Weight (g) ^{a}** | | | **Weight change (g) after 21 days of administrat ion** |
|---|---|---|---|---|---|
| | | **Before administrat ion** | **Day 21 of grouping and administration** | **P^{b}** | |
| G1 | hIgG | 18.1 ± 0.3 | 21.3 ± 0.5 | - | +3.2 |
| G2 | Avelumab | 18.4 ± 0.4 | 21.1 ± 0.4 | 0.765 | +2.7 |
| G3 | Sim-anti-PD-L1 | 18.4 ± 0. 3 | 21.5 ± 0. 5 | 0.747 | +3.1 |

| | | | | | |
|---|---|---|---|---|---|
| Note: a: mean ± standard error; B: the body weight of the administration group and the body weight of the hIgG control were statistically compared on the 21st day of grouping and administration, T-test. | | | | | |

### Example 13 In vivo pharmacodynamics detection of humanized anti-PD-L1 antibody

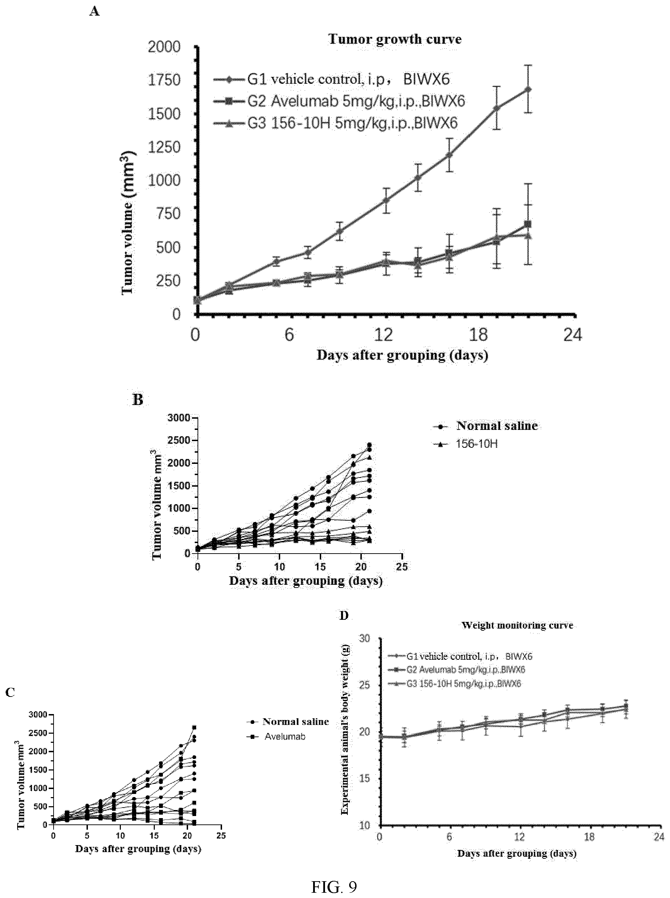

MC38-hPD-L1 cells were inoculated subcutaneously at a concentration of 5 × 10⁵/0.1 mL on the right side of female B-hPD-L1 transgenic mice aged 6-8 weeks (Biocytogen Jiangsu Gene Biotechnology Co., Ltd.). When the tumor grew to about 108 mm³, 24 mice were selected according to the tumor volume and randomly divided into 3 groups with 8 in each group, a total of 3 groups, namely: saline, Avelumab (5 mg/kg), 156-10H (5 mg/kg). All groups were given intraperitoneal injection twice a week for 6 times, and the experiment ended 5 days after the last administration. The body weight and tumor volume of mice were measured three times a week during administration and observation, and the measured values were recorded. The tumor volume (long diameter × short diameter²/2) and the growth inhibition rate (TGI_{TV} (%)) were calculated. On the 21st day of grouping and administration, the positive drug Avelumab group and the PD-L1 antibody 156-10H group had significant and similar inhibitory effects on tumor volume compared with the saline control group, with statistical differences (P < 0.05). See FIGs. 9A, B, C, and Table 10.

**Table 10. Effect of test substance on volume of MC38-hPD-L1 tumor from B-hPD-L1 humanized mouse**

| **Group** | **Test substance** | **Tumor volume (mm³) ^{a}** | | | **P^{b}** |
|---|---|---|---|---|---|
| | | **Before adminis a** | **Day 21 of grouping and t dministration** | **TGI_{TV} (%) ration** | |
| G1 | Normal saline | 108 ± 4 | 1682 ± | - | - |
| | | | 177 | | |
| G2 | Avelumab | 108 ± 5 | 672 ± 301 | 64.2 | ^{∗} 0.012 |
| G3 | 156-10H | 108 ± 5 | 593 ± 223 | 69.2 | ^{∗∗}0.002 |

| | | | | | |
|---|---|---|---|---|---|
| Note: a: mean ± standard error; B: The tumor volume of the administration group and the tumor volume of the normal saline control group were statistically compared on the 21st day of grouping and administration, T-test analysis, ^{∗} P < 0.05, ^{∗ ∗} P < 0.01. | | | | | |

The experimental animals were in good activity and eating state during administration, and their weight increased to a certain extent, as shown in FIG.9D and Table 11.

**Table 11 Effect of test substance on body weight of B-hPD-L1 humanized mouse transplanted with MC38-hPD-L1 cells**

| **Group** | **Test substance** | **Weight (g) ^{a}** | | | **Weight change (g) after 21 day of administration** |
|---|---|---|---|---|---|
| | | **Before administration** | **Day 21 of grouping and administration** | **P^{b}** | |
| G1 | Normal saline | 19.5 ± 0.7 | 22.5 ± 0.9 | - | +3.0 |
| G2 | Avelumab | 19.5 ± 0.6 | 22.8 ± 0.6 | 0.762 | +3.3 |
| G3 | 156-10H | 19.5 ± 0.3 | 22.4 ± 0.5 | 0.972 | +2.9 |

| | | | | | |
|---|---|---|---|---|---|
| Note: a: mean ± standard error; B: The weight of the administration group and the weight of the vehicle control group were statistically compared on the 21st day of grouping and administration, and T-test analysis was performed. | | | | | |

The above results indicate that humanized PD-L1 antibody 156-10H had a significant inhibitory effect on the growth of subcutaneous transplanted tumor MC38-hPD-L1 and showed high safety. As compare with positive control antibody Avelumab, the TGI level of the two antibodies was comparable, and 156-10H showed a more homogeneous anti-tumor effect.

The antibody against human programmed death ligand-1 (PD-L1) and use thereof provided by the present invention have been illustrated in detail above. The principles and implementations of the present invention are described herein by using specific embodiments, and the descriptions of the above embodiments are only used to help understand the method and the core idea of the present invention. It should be pointed out that for those skilled in the art, several improvements and modifications can be made to the present invention without departing from the principle of the present invention, and these improvements and modifications also fall within the protection scope of the claims of the present invention.

## Claims

1. An isolated antibody or antigen-binding fragment thereof specifically binding to human programmed death ligand-1 (PD-L1), wherein the antibody or antigen-binding fragment thereof comprises a combination of heavy chain CDRs and a combination of light chain CDRs:
(1) the combination of heavy chain CDRs comprises CDR1-VH, CDR2-VH and CDR3-VH; the CDR1-VH, CDR2-VH and CDR3-VH have sequences selected from the group consisting of the following combinations or sequences with insertion, deletion and/or substitution of 1, 2, 3 or more amino acids compared to the following combinations:
| Combination No. | SEQ ID NO: | | |
|---|---|---|---|
| | CDR1-VH | CDR2-VH | CDR3-VH |
| VH1 | SEQ ID NO: 19 | SEQ ID NO: 20 | SEQ ID NO: 21 |
| VH2 | SEQ ID NO: 25 | SEQ ID NO: 26 | SEQ ID NO: 27 |
| VH3 | SEQ ID NO: 31 | SEQ ID NO: 32 | SEQ ID NO: 33 |
| VH4 | SEQ ID NO: 37 | SEQ ID NO: 38 | SEQ ID NO: 39 |
| VH5 | SEQ ID NO: 43 | SEQ ID NO: 44 | SEQ ID NO: 45 |
| VH6 | SEQ ID NO: 49 | SEQ ID NO: 50 | SEQ ID NO: 51 |
| VH7 | SEQ ID NO: 55 | SEQ ID NO: 56 | SEQ ID NO: 57 |
| VH8 | SEQ ID NO: 61 | SEQ ID NO: 62 | SEQ ID NO: 63 |
| VH9 | SEQ ID NO: 67 | SEQ ID NO: 68 | SEQ ID NO: 69 |
| VH10 | SEQ ID NO: 73 | SEQ ID NO: 74 | SEQ ID NO: 75 |
| VH11 | SEQ ID NO: 79 | SEQ ID NO: 80 | SEQ ID NO: 81 |
| VH12 | SEQ ID NO: 85 | SEQ ID NO: 86 | SEQ ID NO: 87 |
| VH13 | SEQ ID NO: 91 | SEQ ID NO: 92 | SEQ ID NO: 93 |
| VH14 | SEQ ID NO: 97 | SEQ ID NO: 98 | SEQ ID NO: 99 |
| VH15 | SEQ ID NO: 103 | SEQ ID NO: 104 | SEQ ID NO: 105 |
| VH16 | SEQ ID NO: 109 | SEQ ID NO: 110 | SEQ ID NO: 111 |
| VH17 | SEQ ID NO: 115 | SEQ ID NO: 116 | SEQ ID NO: 117 |
| VH18 | SEQ ID NO: 121 | SEQ ID NO: 122 | SEQ ID NO: 123 |
and
(2) the combination of light chain CDRs comprises CDR1-VL, CDR2-VL and CDR3-VL; the CDR1-VL, CDR2-VL and CDR3-VL have sequences selected from the group consisting of the following combinations or sequences with insertion, deletion and/or substitution of 1, 2, 3 or more amino acids compared to the following combinations:
| Combination No. | SEQ ID NO: | | |
|---|---|---|---|
| | CDR1-VL | CDR2-VL | CDR3-VL |
| VL1 | SEQ ID NO: 22 | SEQ ID NO: 23 | SEQ ID NO: 24 |
| VL2 | SEQ ID NO: 28 | SEQ ID NO: 29 | SEQ ID NO: 30 |
| VL3 | SEQ ID NO: 34 | SEQ ID NO: 35 | SEQ ID NO: 36 |
| VL4 | SEQ ID NO: 40 | SEQ ID NO: 41 | SEQ ID NO: 42 |
| VL5 | SEQ ID NO: 46 | SEQ ID NO: 47 | SEQ ID NO: 48 |
| VL6 | SEQ ID NO: 52 | SEQ ID NO: 53 | SEQ ID NO: 54 |
| VL7 | SEQ ID NO: 58 | SEQ ID NO: 59 | SEQ ID NO: 60 |
| VL8 | SEQ ID NO: 64 | SEQ ID NO: 65 | SEQ ID NO: 66 |
| VL9 | SEQ ID NO: 70 | SEQ ID NO: 71 | SEQ ID NO: 72 |
| VL10 | SEQ ID NO: 76 | SEQ ID NO: 77 | SEQ ID NO: 78 |
| VL11 | SEQ ID NO: 82 | SEQ ID NO: 83 | SEQ ID NO: 84 |
| VL12 | SEQ ID NO: 88 | SEQ ID NO: 89 | SEQ ID NO: 90 |
| VL13 | SEQ ID NO: 94 | SEQ ID NO: 95 | SEQ ID NO: 96 |
| VL14 | SEQ ID NO: 100 | SEQ ID NO: 101 | SEQ ID NO: 102 |
| VL15 | SEQ ID NO: 106 | SEQ ID NO: 107 | SEQ ID NO: 108 |
| VL16 | SEQ ID NO: 112 | SEQ ID NO: 113 | SEQ ID NO: 114 |
| VL17 | SEQ ID NO: 118 | SEQ ID NO: 119 | SEQ ID NO: 120 |
| VL18 | SEQ ID NO: 124 | SEQ ID NO: 125 | SEQ ID NO: 126 |
each of CDR1-VH, CDR2-VH, CDR3-VH, CDR1-VL, CDR2-VL and CDR3-VL is defined by a common analysis method of KABAT, Chothia or IMGT numbering.

2. An isolated humanized antibody or antigen-binding fragment thereof specifically binding to human programmed death ligand-1 (PD-L1), wherein the antibody or antigen-binding fragment thereof comprises a combination of heavy chain CDRs and a combination of light chain CDRs:
(1) the combination of heavy chain CDRs comprises CDR1-VH, CDR2-VH and CDR3-VH; the CDR1-VH, CDR2-VH and CDR3-VH have sequences selected from the group consisting of the following combinations or sequences with insertion, deletion and/or substitution of 1, 2, 3 or more amino acids compared to the following combinations:
| Combination No. | SEQ ID NO: | | |
|---|---|---|---|
| | CDR1-VH | CDR2-VH | CDR3-VH |
| VH19 | SEQ ID NO: 135 | SEQ ID NO: 136 | SEQ ID NO: 137 |
| VH20 | SEQ ID NO: 141 | SEQ ID NO: 142 | SEQ ID NO: 143 |
| VH21 | SEQ ID NO: 147 | SEQ ID NO: 148 | SEQ ID NO: 149 |
| VH22 | SEQ ID NO: 153 | SEQ ID NO: 154 | SEQ ID NO: 155 |
| VH23 | SEQ ID NO: 159 | SEQ ID NO: 160 | SEQ ID NO: 161 |
| VH24 | SEQ ID NO: 165 | SEQ ID NO: 166 | SEQ ID NO: 167 |
| VH25 | SEQ ID NO: 171 | SEQ ID NO: 172 | SEQ ID NO: 173 |
| VH26 | SEQ ID NO: 177 | SEQ ID NO: 178 | SEQ ID NO: 179 |
and
(2) the combination of light chain CDRs comprises CDR1-VL, CDR2-VL and CDR3-VL; the CDR1-VL, CDR2-VL and CDR3-VL have sequences selected from the group consisting of the following combinations or sequences with insertion, deletion and/or substitution of 1, 2, 3 or more amino acids compared to the following combinations:
| Combination No. | SEQ ID NO: | | |
|---|---|---|---|
| | CDR1-VL | CDR2-VL | CDR3-VL |
| VL19 | SEQ ID NO: 138 | SEQ ID NO: 139 | SEQ ID NO: 140 |
| VL20 | SEQ ID NO: 144 | SEQ ID NO: 145 | SEQ ID NO: 146 |
| VL21 | SEQ ID NO: 150 | SEQ ID NO: 151 | SEQ ID NO: 152 |
| VL22 | SEQ ID NO: 156 | SEQ ID NO: 157 | SEQ ID NO: 158 |
| VL23 | SEQ ID NO: 162 | SEQ ID NO: 163 | SEQ ID NO: 164 |
| VL24 | SEQ ID NO: 168 | SEQ ID NO: 169 | SEQ ID NO: 170 |
| VL25 | SEQ ID NO: 174 | SEQ ID NO: 175 | SEQ ID NO: 176 |
| VL26 | SEQ ID NO: 180 | SEQ ID NO: 181 | SEQ ID NO: 182 |
each of CDR1-VH, CDR2-VH, CDR3-VH, CDR1-VL, CDR2-VL and CDR3-VL is defined by a common analysis method of KABAT, Chothia or IMGT numbering.

3. The antibody or antigen-binding fragment thereof according to claim 1 or 2, comprising a combination of heavy chain CDRs and light chain CDRs, wherein the combination of heavy chain CDRs and light chain CDRs is selected from the group consisting of VH1+VL1, VH2+VL2, VH3+VL3, VH4+VL4, VH5+VL5, VH6+VL6, VH7+VL7, VH8+VL8, VH9+VL9, VH10+VL10, VH11+VL11, VH12+VL12, VH13+VL13, VH14+VL14, VH15+VL15, VH16+VL16, VH17+VL17, VH18+VL18, VH19+VL19, VH20+VL20, VH21+VL21, VH22+VL22, VH23+VL23, VH24+VL24, VH25+VL25, VH26+VL26 and CDR combinations having sequences with insertion, deletion and/or substitution of 1, 2, 3 or more amino acids compared to sequences of the combination of heavy chain CDRs and light chain CDRs.

4. The antibody or antigen-binding fragment thereof according to any one of claims 1 to 3, comprising,
1) a heavy chain variable region and a light chain variable region having the sequences set forth in SEQ ID NO: 1 and SEQ ID NO: 2, respectively, or sequences having 70%, 75%, 80%, 85%, 90%, 95%, 96%, 97%, 98%, 99% or higher identity to the aforementioned sequence;
2) a heavy chain variable region and a light chain variable region having the sequences set forth in SEQ ID NO: 3 and SEQ ID NO: 4, respectively, or sequences having 70%, 75%, 80%, 85%, 90%, 95%, 96%, 97%, 98%, 99% or higher identity to the aforementioned sequences;
3) a heavy chain variable region and a light chain variable region having the sequences set forth in SEQ ID NO: 5 and SEQ ID NO: 6, respectively, or sequences having 70%, 75%, 80%, 85%, 90%, 95%, 96%, 97%, 98%, 99% or higher identity to the aforementioned sequences;
4) a heavy chain variable region and a light chain variable region having the sequences set forth in SEQ ID NO: 7 and SEQ ID NO: 8, respectively, or sequences having 70%, 75%, 80%, 85%, 90%, 95%, 96%, 97%, 98%, 99% or higher identity to the aforementioned sequences;
5) a heavy chain variable region and a light chain variable region having the sequences set forth in SEQ ID NO: 9 and SEQ ID NO: 10, respectively, or sequences having 70%, 75%, 80%, 85%, 90%, 95%, 96%, 97%, 98%, 99% or higher identity to the aforementioned sequences;
6) a heavy chain variable region and a light chain variable region having the sequences set forth in SEQ ID NO: 11 and SEQ ID NO: 12, respectively, or sequences having 70%, 75%, 80%, 85%, 90%, 95%, 96%, 97%, 98%, 99% or higher identity with to the aforementioned sequences;
7) a heavy chain variable region and a light chain variable region having the sequences set forth in SEQ ID NO: 13 and SEQ ID NO: 14, respectively, or sequences having 70%, 75%, 80%, 85%, 90%, 95%, 96%, 97%, 98%, 99% or higher identity to the aforementioned sequences;
8) a heavy chain variable region and a light chain variable region having the sequences set forth in SEQ ID NO: 15 and SEQ ID NO: 16, respectively, or sequences having 70%, 75%, 80%, 85%, 90%, 95%, 96%, 97%, 98%, 99% or higher identity to the aforementioned sequences;
9) a heavy chain variable region and a light chain variable region having the sequences set forth in SEQ ID NO: 17 and SEQ ID NO: 18, respectively, or sequences having 70%, 75%, 80%, 85%, 90%, 95%, 96%, 97%, 98%, 99% or higher identity to the aforementioned sequences;
10) a heavy chain variable region and a light chain variable region having the sequences set forth in SEQ ID NO: 127 and SEQ ID NO: 128, respectively, or sequences having 70%, 75%, 80%, 85%, 90%, 95%, 96%, 97%, 98%, 99% or higher identity with to the aforementioned sequences;
11) a heavy chain variable region and a light chain variable region having the sequences set forth in SEQ ID NO: 129 and SEQ ID NO: 130, respectively, or sequences having 70%, 75%, 80%, 85%, 90%, 95%, 96%, 97%, 98%, 99% or higher identity with to the aforementioned sequences;
12) a heavy chain variable region and a light chain variable region having the sequences set forth in SEQ ID NO: 131 and SEQ ID NO: 132, respectively, or sequences having 70%, 75%, 80%, 85%, 90%, 95%, 96%, 97%, 98%, 99% or higher identity with to the aforementioned sequences; or
13) a heavy chain variable region and a light chain variable region having the sequences set forth in SEQ ID NO: 133 and SEQ ID NO: 134, respectively, or sequences having 70%, 75%, 80%, 85%, 90%, 95%, 96%, 97%, 98%, 99% or higher identity to the aforementioned sequences.

5. The antibody or antigen-binding fragment thereof according to any one of claims 1 to 4, wherein the antibody or antigen-binding fragment thereof has a dissociation constant (KD) of no more than 10 nM for binding to human programmed death ligand-1 (PD-L1) and a dissociation constant (KD) of no more than 100 nM for binding to cynomolgus monkey programmed death ligand-1 (PD-L1).

6. The antibody or antigen-binding fragment thereof according to any one of claims 1 to 5, wherein the antibody or antigen-binding fragment thereof is:
(1) a chimeric antibody or fragment thereof;
(2) a humanized antibody or fragment thereof; or
(3) a fully humanized antibody or fragment thereof.

7. The antibody or antigen-binding fragment thereof according to any one of claims 1 to 6, wherein the antibody comprises a constant region selected from the group consisting of human or murine IgG1, IgG2, IgG3, IgG4, IgA, IgM, IgE and IgD; preferably a constant region selected from the group consisting of human or murine IgG1, IgG2, IgG3 and IgG4.

8. The antibody or antigen-binding fragment thereof according to any one of claims 1 to 7, wherein the antigen-binding fragment thereof is selected from the group consisting of F(ab)₂, Fab', Fab, Fv, scFv, bispecific antibody, nanobody, a minimum recognition unit of an antibody and a combination thereof.

9. An antibody or antigen-binding fragment thereof, wherein the antibody or antigen-binding fragment thereof competitively binds to PD-L1 or an epitope thereof with the antibody or antigen-binding fragment thereof according to any one of claims 1 to 8, and has characteristics of:
1) specifically binding to a PD-L1 recombinant protein and a cell expressing PD-L1;
2) blocking the binding of PD-L1 to PD-1 protein;
3) suppressing the binding of PD-1 to PD-L1 expressed on cell surface;
4) enhancing the activity of T cells;
5) mediating antibody-dependent cell-mediated cytotoxicity (ADCC) activity; or/and
6) inhibiting tumor growth.

10. An isolated nucleic acid encoding the antibody or antigen-binding fragment thereof according to any one of claims 1 to 9, or a combination thereof.

11. An expression vector comprising the isolated nucleic acid according to claim 10.

12. An isolated host cell comprising the isolated nucleic acid according to claim 10 or the expression vector according to claim 11; preferably, the host cell is a eukaryotic cell or a prokaryotic cell; more preferably, the host cell is derived from mammalian cells, yeast cells, insect cells, Escherichia coli and/or Bacillus subtilis; more preferably, the host cell is Chinese Hamster Ovary (CHO) cells.

13. A method for producing an antibody or antigen-binding fragment thereof, comprising culturing the host cell according to claim 12 under a suitable condition and isolating the antibody or antigen-binding fragment thereof.

14. A pharmaceutical composition comprising the antibody or antigen-binding fragment thereof according to any one of claims 1 to 9, the isolated nucleic acid according to claim 10, the expression vector according to claim 11, the host cell according to claim 12, or the antibody or antigen-binding fragment thereof produced by the method according to claim 13, and a pharmaceutically acceptable carrier; preferably, the pharmaceutical composition further comprises an additional anti-tumor agent.

15. Use of the antibody or antigen-binding fragment thereof according to any one of claim 1 to 9, the isolated nucleic acid molecule according to claim 10, the expression vector according to claim 11, the host cell according to claim 12, the antibody or antigen-binding fragment thereof produced by the method according to claim 13, or the pharmaceutical composition according to claim 14 in the manufacture of a medicament for the prevention and/or treatment of a disease associated with abnormal expression of PD-L1, wherein the disease is preferably a tumor.

16. A method for preventing and/or treating a disease associate with abnormal expression of PD-L1 and/or abnormal T cell function, comprising administering to a subject in need thereof the antibody or antigen-binding fragment thereof according to any one of claims 1 to 9, the isolated nucleic acid according to claim 10, the expression vector according to claim 11, the host cell according to claim 12, the antibody or antigen-binding fragment thereof produced by the method according to claim 13, or the pharmaceutical composition according to claim 14, wherein the disease is preferably a tumor, and the tumor is preferably colorectal cancer.

17. A kit comprising the antibody or antigen-binding fragment thereof according to any one of claim 1 to 9, the isolated nucleic acid molecule according to claim 10, the expression vector according to claim 11, the host cell according to claim 12, the antibody or antigen-binding fragment thereof produced by the method according to claim 13, and an instruction for use.
